(19) Europäisches Patentamt
European Patent Office
Office européen des brevets

(11) Publication number: **0 286 364 B1**

(12) # EUROPEAN PATENT SPECIFICATION

(45) Date of publication of patent specification: **13.07.94**

(51) Int. Cl.⁵: **C07C 233/75**, C07C 235/46, C07C 255/49, C07C 255/57, C07D 257/04, A61K 31/165, A61K 31/41

(21) Application number: **88303037.1**

(22) Date of filing: **06.04.88**

(54) **Substituted Di-t-Butylphenols.**

(30) Priority: **06.04.87 US 34537**

(43) Date of publication of application:
**12.10.88 Bulletin 88/41**

(45) Publication of the grant of the patent:
**13.07.94 Bulletin 94/28**

(84) Designated Contracting States:
**BE CH DE ES FR GB IT LI NL SE**

(56) References cited:
**EP-A- 0 211 549** **DE-A- 2 021 133**
**DE-A- 3 521 558** **FR-A- 2 035 771**
**US-A- 3 487 099** **US-A- 4 128 664**

**PATENT ABSTRACTS OF JAPAN, unexamined applications, C section, vol. 3, no. 147, December 5, 1979, THE PATENT OFFICE JAPANESE GOVERNMENT page 100 C 66**

(73) Proprietor: **RIKER LABORATORIES, INC.**
**3M Center - 225-1N-07**
**Saint Paul Minnesota 55144-1000(US)**

(72) Inventor: **Scherrer, Robert A., c/o Riker Labs. Inc.,**
**225-1N-07, St. Paul**
**Minnesota 55144,(US)**
Inventor: **Rustad, Mark A., c/o Riker Labs. Inc.,**
**225-1N-07 St. Paul,**
**Minnesota 55144,(US)**

(74) Representative: **Baillie, Iain Cameron et al**
**c/o Ladas & Parry**
**Altheimer Eck 2**
**D-80331 München (DE)**

**EP 0 286 364 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to substituted di-t-butylphenols which are inhibitors of leukotriene synthesis. This invention further relates to pharmaceutical compositions containing such compounds, pharmacological methods for using such compounds and synthetic intermediates for preparing such compounds.

### Background of the Invention

The leukotrienes are a group of biologically active mediators derived from arachidonic acid through the action of lipoxygenase enzyme systems. The leukotrienes are potent smooth muscle contracting agents, particularly on respiratory smooth muscle, but also on other tissues as well. In addition, they promote mucous production, modulate vascular permeability changes and are potent inflammatory mediators in human skin. There are two groups of leukotrienes derived from the common unstable precursor Leukotriene $A_4$. The first of these are the peptido-lipid leukotrienes, the most important being Leukotrienes $C_4$ and $D_4$. These compounds collectively account for the biologically active material known as the slow reacting substance of anaphylaxis ("SRS-A").

The most important compound in the second group of leukotrienes, namely dihydroxy fatty acids, is Leukotriene $B_4$. This compound is a potent chemotactic agent for neutrophils and eosinophils and, in addition, may modulate a number of other functions of these cells. It also affects other cell types such as lymphocytes and, for example, may modulate the action of suppressor cells and natural killer cells. When injected *in vivo*, in addition to promoting the accumulation of leukocytes, Leukotriene $B_4$ is also a potent hyperalgesic agent and can modulate vascular permeability changes through a neutrophil dependent mechanism. Both groups of leukotrienes are formed following oxygenation of arachidonic acid through the action of a lipoxygenase enzyme. See, for example, D. M. Bailey *et al.*, *Ann. Rpts. Med. Chem.*, *17*, 203 (1982).

### RESPIRATORY CONDITIONS

*Asthma*. The leukotrienes are potent spasmogens of human trachea, bronchus, and lung parenchymal strip, and when administered to normal volunteers as aerosols are 3,800 times more potent than histamine at inducing a 50% decrease in air flow at 30% of vital capacity. They mediate increases in vascular permeability in animals and promote mucous production in human bronchial explants. In addition, Leukotriene $B_4$ may also mediate mucous production and could be an important mediator of neutrophil and eosinophil accumulation in asthmatic lungs. Lipoxygenase products are also thought to be regulators of mast cell degranulation, and recent studies with human lung mast cells have suggested that lipoxygenase inhibitors (but not corticosteroids), may suppress antigen-induced mast cell degranulation. *In vitro* studies have shown that antigen challenge of human lung results in the release of leukotrienes and that, in addition, purified human mast cells can produce substantial amounts of leukotrienes. There is, therefore, good evidence that the leukotrienes are important mediators of human asthma. Lipoxygenase inhibitors would therefore be a new class of drugs for the treatment of asthma. See, for example, B. Samuelsson, *Science*, *220*, 568-575 (1983).

### SKIN DISEASES

*Psoriasis*. Psoriasis is a human skin disease which affects between two and six percent of the population. There is no adequate therapy for psoriasis and related skin conditions. The evidence for leukotriene involvement in these diseases is as follows. One of the earliest events in the development of prepapillary lesions is the recruitment of leukocytes to the skin site. Injection of Leukotriene $B_4$ into human skin results in a pronounced neutrophil accumulation. There are gross abnormalities in arachidonic acid metabolism in human psoriatic skin. In particular, highly elevated levels of free arachidonic acid can be measured as well as large amounts of lipoxygenase products. Leukotriene $B_4$ has been detected in psoriatic lesions, but not in non-involved skin, in biologically significant amounts.

### ALLERGIC CONDITIONS

Leukotrienes can be measured in nasal washings from patients with allergic rhinitis and are greatly elevated following antigen challenge. Leukotrienes may mediate this disease through their ability to regulate mast cell degranulation, to modulate mucous production and mucocillary clearance, and to mediate the

accumulation of inflammatory leukocytes.

Leukotrienes may also mediate other diseases. These include atopic dermatitis, gouty arthritis, gall bladder spasms and ulcerative colitis. In addition, they may have a role in cardiovascular disease because Leukotrienes $C_4$ and $D_4$ act as coronary and cerebral arterial vasoconstrictors and these compounds may also have negative inotropic effects on the myocardium. In addition, the leukotrienes are important mediators of inflammatory disease through their ability to modulate leukocyte and lymphocyte function.

Many substituted di-t-butylphenols are known. Generally these compounds may be useful as antioxidants. Some of these compounds are also known to be active antiinflammatory agents.

US-A-4,128,664 discloses certain 2,6-di-t-butylphenols substituted in the 4 position by an N-substituted carbamyl or by an N-substituted acylamino group. These compounds are stated as useful as anti-inflammatory agents.

EP-A-0,211,549 discloses certain 2,6-di-t-butylphenols substituted in the 4 position by an anilino group which is substituted by a group which includes carboxyl, tetrazolyl or N-methyltetrazolyl. These components are stated to be useful as inhibitors of leukotriene biosynthesis and as antiallergic agents.

## Detailed Description of the Invention

The present invention relates to compounds of Formula I:

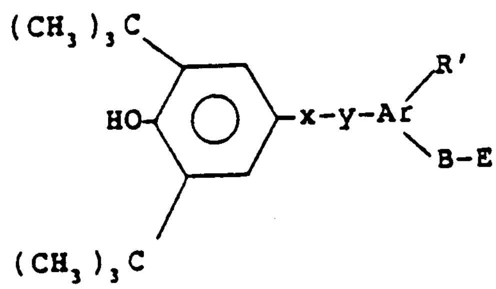

(I)

wherein x is carbonyl or N-R, and y is carbonyl or N-R; with the proviso that when x is carbonyl, y is N-R, and when x is N-R, y is carbonyl; further provided that:

1) when x is N-R,

R is hydrogen,

R' is hydrogen,

B is a carbon-carbon bond,

Ar is phenyl or cyclohexyl, and

E is tetrazolyl or carboxyl,

with the proviso that when Ar is cyclohexyl, E is carboxyl and is alpha to the carbonyl-cyclohexyl bond; and

2) when x is carbonyl,

R is hydrogen or lower alkyl,

R' is hydrogen, halogen, trifluoromethyl, lower alkyl or lower alkoxy,

Ar is phenyl, E is carboxyl, tetrazolyl, or N-methyltetrazolyl, and

B is as follows:

when E is carboxyl, B is a carbon-carbon bond; an alkylene group containing one to seven carbon atoms; or an alkylene group containing one to seven carbon atoms and being either (a) interrupted by an ether linkage, a thioether linkage or a carbon-carbon double bond, or (b) attached to the phenyl ring by an oxygen atom or an unoxidized sulfur atom; or

when E is tetrazolyl or N-methyltetrazolyl, B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom or carboxamido;

and a derivative of the compound wherein E is carboxyl, selected from lower alkyl esters, (lower)alkylamino-(lower)alkyl esters, esters of glycolamide, pharmaceutically acceptable (lower)alkylamino(lower)alkyl ester acid-addition salts and pharmaceutically acceptable carboxylate salts;

and a derivative of the compound wherein E is tetrazolyl, selected from pharmaceutically acceptable alkali

metal and alkaline earth salts of the tetrazolyl moiety.

Compounds of Formula I are useful to inhibit bronchoconstriction due to allergic response and to inhibit leukotriene synthesis

The present invention also provides novel compounds of Formula II below

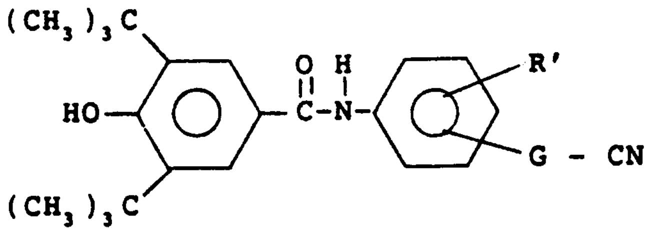

II

wherein

R′ is hydrogen, halogen, trifluoromethyl, lower alkyl or lower alkoxy; and

G is a carbon-carbon bond or alkylene of one to about four carbon atoms.

Furthermore, the present invention also provides novel compounds of Formula III below

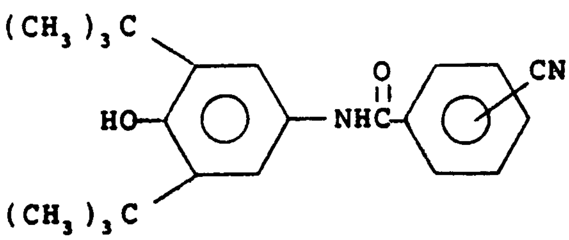

III

Compounds of Formula II and III are useful synthetic intermediates for preparing certain of the compounds of Formula I.

The preferred compounds of Formula I of the invention are those wherein B is a carbon-carbon bond.

Another preferred subset of compounds of Formula I are those wherein X is carbonyl.

When R is lower alkyl it is presently preferred to be methyl. The presently preferred R group is hydrogen.

By "lower" as used in connection with "alkyl" is meant that such groups contain one to four carbon atoms. Most preferred alkyl groups contain one to two carbon atoms. By "lower" as used in connection with "alkylene" is meant that such groups contain two to four carbon atoms.

In the compounds of Formula I wherein E is tetrazolyl, two tautomeric forms of tetrazolyl exist as is known to those skilled in the art. Tautomerism does not exist in tetrazolyl moieties where the tetrazolyl ring is substituted on a nitrogen atom by methyl. Instead, two N-methyl isomers are obtained, one in which the methyl group is in the 1-position, the other in which it is in the 2-position. All such tautomers and isomers are within the scope of this invention.

It is well known to the art that pharmaceutically acceptable salts such as alkali metal, alkaline earth, aluminum and other metal and amine salts of pharmaceutically active acids are the equivalents of the acids in terms of activity, and in some cases may even offer advantages in absorption and formulation. Pharmaceutically-acceptable carboxylate salts of the compounds of the invention which contain carboxyl as E are prepared in an inert atmosphere by reaction of the acid with a base and subsequent evaporation to dryness, preferably under mild conditions. The base may be organic, e.g., sodium methoxide or an amino, or inorganic, e.g., sodium hydroxide. Alternatively, the cation of a carboxylate salt, e.g., sodium, may be displaced by a second cation such as calcium or magnesium when the salt of the second cation is more insoluble in a selected solvent.

Other useful derivatives of the compounds of the invention which contain carboxyl as E include alkyl esters, alkylaminoalkyl esters, and salts of the latter. In the ester derivatives, the hydrogen portion of the carboxylic acid group is replaced with an alkyl or substituted alkyl, preferably an alkylaminoalkyl group.

Esters of the compounds of the invention may be obtained as intermediates during the preparation of the corresponding acid. In some cases, the esters may be prepared directly using standard synthetic methods. These esters may exhibit antiallergic activity, but they are primarily of interest as synthetic intermediates, although in some instances hydrolyzable or salt-forming esters may be of interest as

therapeutic agents.

Ester derivatives may be obtained by alkylation of an alkali metal salt of the compound in dimethylformamide with an alkyl iodide or dialkylaminoalkylchloride, or by starting with esters instead of acids in Reaction Scheme A below.

Pharmaceutically acceptable alkali metal and alkaline earth salts may also be prepared of compounds of Formula I wherein E is tetrazolyl by methods known to those skilled in the art.

Compounds of Formula I wherein x is carbonyl, y is N-R, and E is -COOH (with R, R′, Ar and B being as defined previously in the context of Formula I) may be prepared in accordance with the procedures of Reaction Scheme A below.

**REACTION SCHEME A**

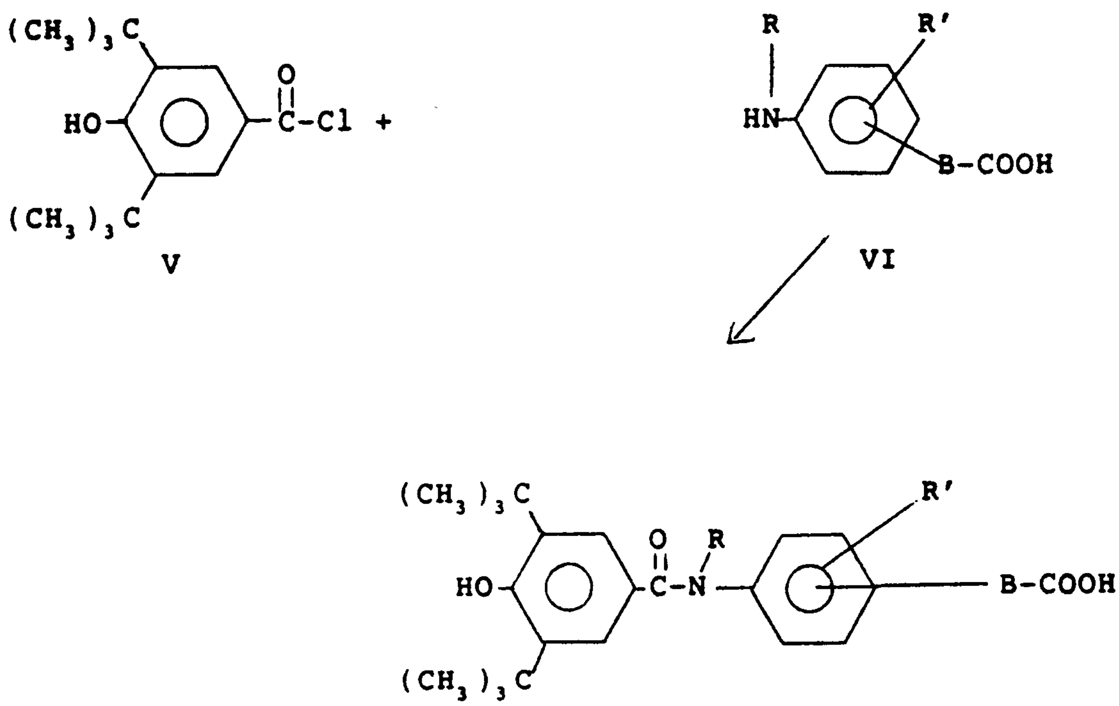

V

VI

VII

In Reaction Scheme A, known 3,5-di-t-butyl-4-hydroxybenzoyl chloride (V) is reacted with an amine of Formula VI to provide a compound of Formula VII which is a subgenus of Formula I. The reaction is conducted by combining the 3,5-di-t-butyl-4-hydroxybenzoyl chloride with 2 to 2.5 equivalents of an amine of Formula VI in an inert solvent such as 1,2-dimethoxyethane, accompanied by gentle heating if necessary. Amines of Formula VI are known compounds or may be prepared using known methods. Examples of amines of Formula VI are m-aminobenzoic acid, p-aminobenzoic acid, o-aminobenzoic acid (anthranilic acid), p-aminophenyl acetic acid and the like.

The product of Formula VII may be readily isolated and purified, for example, by recrystallization.

Compounds of Formula I wherein x is carbonyl, y is N-R, and E is tetrazolyl (with R, R′, Ar and B being as defined previously in the context of Formula I) may be prepared in accordance with the procedures of Reaction Scheme B below.

**REACTION SCHEME B**

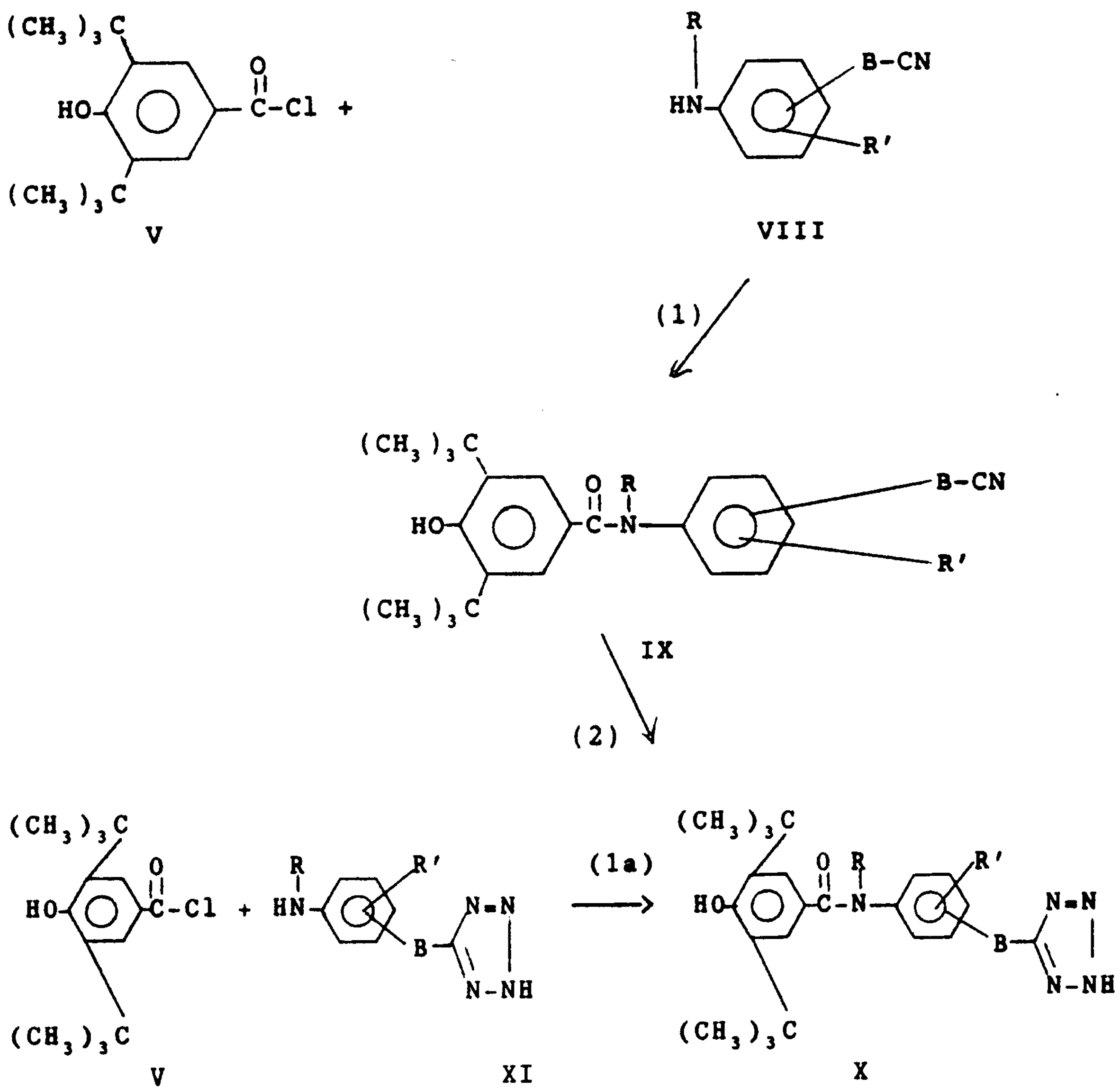


V VIII IX V XI X

In step (1) of Reaction Scheme B, 3,5-di-t-butyl-4-hydroxybenzoyl chloride (V) is reacted with 2 to 2.5 equivalents of an amino nitrile of Formula VIII in the presence of an inert solvent such as 1,2-dimethoxyethane to provide a novel intermediate of Formula IX. Amino nitriles of Formula VIII are known compounds or may be prepared using known methods. Examples of amino nitriles of Formula VIII are o-aminobenzonitrile, p-aminobenzonitrile and the like.

In step (2), the intermediate of Formula IX is combined with 3.0 to 5.0 equivalents of sodium azide, 3.0 to 5.0 equivalents of ammonium chloride and 1.0 to 1.7 equivalents of lithium chloride, in the presence of a polar solvent such as N,N-dimethylformamide. The reaction mixture is heated at reflux until thin layer chromatography indicates that the reaction is complete. The product of Formula X, which is a subgenus of Formula I, is readily isolated and may be purified, for example, by recrystallization from a polar solvent.

Hydrolysis of the intermediate of Formula IX using conventional methods may also be undertaken to provide the corresponding compounds of Formula I wherein E is -COOH. Conditions are chosen to afford a preferential hydrolysis of the CN group over the -CONH- functionality using mild conditions known to those skilled in the art.

In alternative step (1a) of Reaction Scheme B, 3,5-di-t-butyl-4-hydroxybenzoyl chloride (V) is combined with 2 to 2.5 equivalents of an amine of Formula XI in the presence of an inert solvent such as 1,2-dimethoxyethane. The product of Formula X again may be readily isolated and purified. Amines of Formula XI are known compounds or may be prepared using conventional methods. Examples of amines of Formula

XI are 3-(1H-tetrazol-5-yl)benzeneamine, and 4-(1H-tetrazol-5-yl)benzeneamine.

Compounds of Formula I wherein x is carbonyl, y is N-R, Ar is phenyl and E is N-methyltetrazolyl (with R, R', and B being as defined previously) may be prepared by alkylating an alkali metal salt of the corresponding compound of Formula I, wherein E is tetrazolyl, with methyl iodide.

Compounds of Formula I wherein Ar is phenyl, x is N-H, y is carbonyl, and E is carboxyl (with R, R' and B being as defined previously in the context of Formula I) may be prepared in accordance with the procedure of Reaction Scheme C below.

**REACTION SCHEME C**

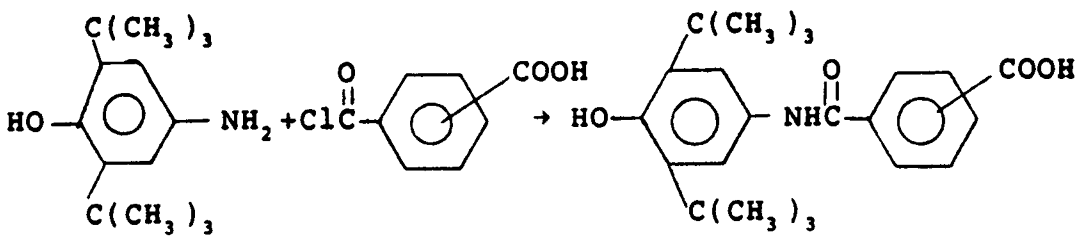

XII XIII XIV

In Reaction Scheme C, known 4-amino-2,6-di-t-butylphenol (XII) is reacted with an acid chloride of formula XIII to provide a compound of Formula XIV which is a subgenus of Formula I. The reaction is conducted by combining the 4-amino-2,6-di-t-butylphenol with an acid chloride of Formula XIII in an inert solvent such as methylene chloride in the presence of triethylamine. Examples of acid chlorides of Formula XIII are m-(chloroformyl)benzoic acid and p-(chloroformyl)benzoic acid. Alternatively, a derivative of the acyl chloride of Formula XIII, wherein the carboxylic acid is esterified, may be reacted with 4-amino-2,6-di-t-butylphenol (XII). Hydrolysis of the product provides a compound of Formula XIV. The product of Formula XIV may be readily isolated and purified using standard techniques.

Compounds of Formula I wherein x is N-R, y is carbonyl, E is carboxyl (with R, R′, B and Ar being as defined previously in the context of Formula I) and the carbonyl and carboxyl bonds are alpha to one another may be prepared in accordance with the procedure of Reaction Scheme D below.

**REACTION SCHEME D**

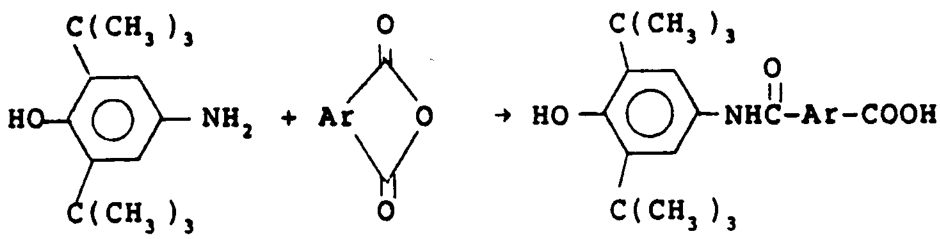

XII XV XVI

In reaction Scheme D, known 4-amino-2,6-di-t-butylphenol (XII) is reacted with an anhydride of Formula XV to provide a compound of Formula XVI which is a subgenus of Formula I. The reaction is conducted by combining the 4-amino-2,6-di-t-butylphenol with one equivalent of an anhydride of Formula XV in a solvent such as diethyl ether or 1,2-dimethoxyethane. Examples of anhydrides of Formula XV are phthalic anhydride, cis-1,2-cyclohexanedicarboxylic anhydride and trans-1,2-cyclohexanedicarboxylic anhydride. The product of Formula XVI may be readily isolated and purified, for example, by recrystallization.

Compounds of Formula I wherein x is N-R, y is carbonyl, Ar is phenyl and E is tetrazolyl (with R, R′ and B being as defined previously in the context of Formula I) may be prepared in accordance with the

procedures of Reaction Scheme E below.

**REACTION SCHEME E**

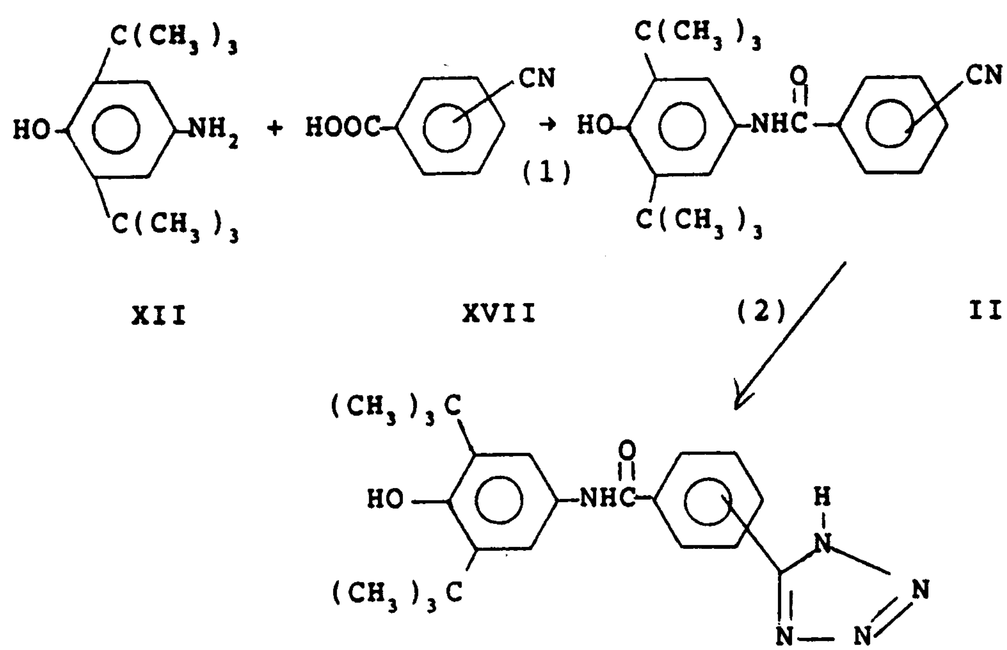


XII XVII III

XVIII

In step (1) of Reaction Scheme E, 4-amino-2,6-di-t-butylphenol (XII) is reacted with one equivalent of a cyanobenzoic acid of Formula XVII in the presence of dicyclohexylcarbodiimide in a solvent such as tetrahydrofuran to provide a novel intermediate of Formula III. Examples of cyanobenzoic acids of Formula XVII are 3-cyanobenzoic acid and 4-cyanobenzoic acid. Alternatively, compounds of Formula III may be prepared using cyanobenzoyl chlorides in a process analogous to that shown in Reaction Scheme C.

In step (2), the intermediate of Formula III is combined with three equivalents of sodium azide, three equivalents of ammonium chloride and one equivalent of lithium chloride in the presence of a polar solvent such as N,N-dimethylformamide. The reaction mixture is heated at about 90 °C until thin layer chromatography indicates that the reaction is complete. The product of Formula XVIII, which is a subgenus of Formula I, is readily isolated and may be purified, for example, by recrystallization.

The activity of the compounds of Formula I may be demonstrated readily by in vivo testing. The in vivo test used to demonstrate antiallergic activity of the compounds of Formula I may be any of those known to those skilled in the art. Preferably, bronchoconstriction in sensitized guinea pigs is measured upon antigen challenge. This test is described in broad terms by Piechuta et al., Immunology, 38, 385 (1979), and more specifically by Hammerbeck and Swingle, Int. Archs. Allergy Appl. Immun. 74, 84-90 (1984). It is used in a modified form as follows: Male Hartley guinea pigs (250-600g) are pretreated with an antihistamine, for example, chlorpheniramine, and then dosed intraperitoneally with a compound of the invention at a level of about 1 to 40 mg/kg 15 minutes prior to challenge. The animals are placed under an inverted dessicator jar (18 x 14 cm) with a constant flow of air coming into the chamber from a compressed-air source to prevent hypoxia, and are aerosol-challenged with either water, or with ovalbumin at a concentration of 10 mg per ml. Air flow leaving the chamber and fluctuations due to respiration are monitored through a separate outlet with a Fleisch No. 0000 pneumotachograph (available from Beckman Instruments, Inc. Schiller Park, IL.) coupled to a Beckman Type R dynograph (available from Beckman Instruments, Inc.). Aerosolization through a third outlet is made via a No. 4 DeVilbiss nebulizer (available from the DeVilbiss Company, Somerset, PA) for 90 seconds at 150 mm Hg. The characteristic respiratory patterns observed are summations of two air exchange processes occurring simultaneously in the chamber. One exchange process is due to inspiration and expiration of air into and out of the animal, while the other exchange process is due to the air flow into and out of the chamber due to respiratory movements. The tracing obtained is the mechanical representation of the summation of those flows. Superimposed on the tracings is a characteristic spiking ("notching"),

which appears to be an exaggerated expiratory movement, the frequency of which correlates with the severity of the bronchoconstrictive reaction. The frequency of notching for 15-minute periods beginning 4 minutes after the beginning of the aerosol challenge is used for comparing various treatments. Effects are considered significant if the t value achieves $p<0.05$. The tested compounds of Formula I exhibit an intraperitoneal $ED_{40}$ of 100 mg per kg or less when tested in the above model. Preferred compounds exhibit an $ED_{40}$ of 50 mg per kg or less and most preferred compounds are active at 25 mg per kg or less.

The oral activity of compounds of Formula I may be demonsrated using the Konzett-Rossler in vivo test method. The activity is determined according to the procedure which follows. The Konzett-Rossler technique (H.Konzett and R. Rossler, Naunyn-Schmiedbergs Arch. Pharmakol., 195, 71-74, 1940 is used to assess the effect of compounds of Formula I of the invention on antigen challenge of male Hartley strain guinea pigs (350-500g). Fourteen days after sensitization with ovalbumin (50 mg/kg intraperitoneally) guinea pigs are anesthetized with pentobarbital (70 mg/kg intraperitoneally) and spontaneous respiration is eliminated with succinylcholine (2 mg/kg intra-peritoneally). The trachea is cannulated and respiration is maintained under positive pressure with a minature ventilator (5 ml/breath, 87 breaths/minute, 10 cm water). Bronchoconstrictor responses are represented as increased excursions of the tracing on a physiological recorder of air overflow to the lungs measured by a pneumotachograph in series with a differential pressure transducer. The guinea pigs are pretreated with an antihistamine, for example, chlorpheniramine, and are then dosed orally at a level of about 5 to 40 mg/kg with a suspension of a compound of Formula I of the invention in 4% aqueous acacia. The animals are challenged with ovalbumin (300 µg/kg intravenously) thirty (30) minutes later.

The compound of Example 7 demonstrated good oral activity in the test method described above.

The compounds may also be tested in more specific tests for the inhibition of leukotriene synthesis. Active compounds are those which exhibit an $IC_{50}$ of 100 micromolar or less, and preferably less than 25 micromolar. Most preferred compounds exhibit an $IC_{50}$ of 10 micromolar or less. The compounds are tested in either intact cells or in cell sonicate. The intact cell assay is similar to that described by Verhagen et al., FEBS Letter 168, 23-28 (1984). Human leukocytes are prepared using standard procedures. The cells are incubated in pH 7.4 Tris buffer containing 5 millimolar calcium chloride and 5 millimolar glutathione. After vehicle or drug incubation, the cells are activated with the calcium ionophore A 23187 (4 micrograms per ml.) After 15 minutes at room temperature, the cells are centrifuged and the supernatants are stored for assay of $LTC_4$ content by radioimmunoassay. The cell sonicate assay utilizes the cell free leukotriene biosynthesis system of M. Steinhoff et al., Biochim. Biophy. Acta., 68, 28 (1980) which consists of homogenized rat basophil leukemia cells. Leukotriene synthesis is initiated by the addition of arachidonate. Solutions are centrifuged and supernatants assayed using a radioimmunoassay developed as described by Aeringhaus et al., FEBS Letter 146, 111-114. Drugs are dissolved in ethanol or dimethyl sulfoxide and preincubated for five minutes. Phenidone is used as a positive control.

Thus, compounds of Formula I are antiallergic agents exhibiting in vivo activity in mammals. The pharmaceutical compositions of the present invention will contain sufficient compound of Formula I in a dosage form suitable for inhibiting the mammalian biosynthesis of leukotrienes, or for the treatment desired. The effective concentration of the Formula I compound in the composition will vary as required by the mode of administration, dosage form, and pharmacological effect and level desired.

For treating pulmonary conditions such as asthma, the mode of administration may be oral, parenteral, by inhalation and by suppository. Suitable oral dosage forms are tablets, elixirs, emulsions, solutions or capsules, including delayed or sustained release dosage forms. Dosage forms for administration by inhalation include aerosols and sprays and will be administered in metered doses if desired.

For treating other allergies or allergic reactions, the compound of Formula I may be administered by any conventional mode, for example, orally, parenterally, topically, subcutaneously, by inhalation and the like. The oral and parenteral dosage forms are as described for pulmonary treatment. The topical application dosage forms include ointments, sprays, controlled release patches, powders and solutions.

For treating inflammation, the mode of administration may be oral, parenteral, by suppository and the like. The various dosage forms are as described above.

For treating skin diseases such as psoriasis, atopic dermatitis and the like, oral, topical or parenteral administration is useful. For topical application to the diseased area salves, patches, controlled release patches, emulsions, etc. are convenient dosage forms.

For treating cardiovascular conditions any suitable mode of administration such as oral or parenteral may be used.

In addition to the common dosage forms listed above, the compounds of Formula I may also be administered for various utilities and indications or for inhibiting leukotriene synthesis by conventional controlled release means and/or delivery devices.

In preparing suitable dosage forms, conventional compounding procedures and ingredients, for example, diluents and carriers may be used. Examples of suitable solid carriers are lactose, terra alba, sucrose, talc, gelatin, agar, pectin, acacia, magnesium stearate, and stearic acid. Examples of suitable liquid carriers are syrup, peanut oil, olive oil, water and a polyethylene glycol such as "PEG 400" (available from union Carbide).

Similarly, the carrier or diluent can include any time delay material well known to the art, such as glyceryl monostearate or glyceryl distearate, these being useful alone or, for example, in combination with wax.

The following examples are provided to illustrate the invention, but are not intended to be limiting thereof.

**EXAMPLE 1**

Preparation of N-(3-Carboxyphenyl)-3,5-Di-t-Butyl-4-Hydroxybenzamide

A solution of 8.6g (0.063 mole) of m-aminobenzoic acid in 200 ml of 1,2-dimethoxyethane was mixed with a suspension of 8.0g (0.0298 mole) of 3,5-di-t-butyl-4-hydroxybenzoyl chloride in 100 ml of 1,2-dimethoxyethane, and the mixture was stirred at 25°C for one hour. The reaction mixture was then filtered. The filtrate was thereafter diluted with 300 ml of water and cooled to give the crude product as a white solid. This material was recrystallized from a mixture of ethanol and water to give 5.6g of white solid N-(3-carboxyphenyl)-3,5-di-t-butyl-4-hydroxybenzamide, m.p. 279-280°C. Analysis: Calculated for $C_{22}H_{27}NO_4$: %C, 71.5; %H, 7.4; %N, 3.8; Found: %C, 72.0; %H, 7.3; %N, 3.7.

**EXAMPLE 2**

Preparation of N-(4-Carboxyphenyl)-3,5-Di-t-Butyl-4-Hydroxybenzamide

A solution of 8.6g (0.063 mole) of p-aminobenzoic acid in 100 ml of 1,2-dimethoxyethane was added to a suspension of 8.0g (0.0298 mole) of 3,5-di-t-butyl-4-hydroxybenzoyl chloride in 100 ml of 1,2-dimethoxyethane, and the mixture was stirred at room temperature for 16 hours. The reaction mixture was then filtered. The filtrate was thereafter diluted with 200 ml of water and chilled. The resulting precipitate was collected, and was then recrystallized from a mixture of ethanol and water to give 3.6g of white needles of N-(4-carboxyphenyl)-3,5-di-t-butyl-4-hydroxybenzamide, m.p. 265-266°C. Analysis: Calculated for $C_{22}H_{27}NO_4$: %C, 71.5; %H, 7.4; %N, 3.8; Found: %C, 71.7; %H, 7.3; %N, 3.6.

**EXAMPLE 3**

Preparation of N-(2-Carboxyphenyl)-3,5-Di-t-Butyl-4-Hydroxybenzamide

A solution of 8.6g (0.063 mole) of anthranilic acid in 50 ml of 1,2-dimethoxyethane was added to a suspension of 8.0g (0.0298 mole) of 3,5-di-t-butyl-4-hydroxybenzoyl chloride in 150 ml of 1,2-dimethoxyethane. The reaction mixture was heated at approximately 75°C for 30 minutes after which time it was stirred for 48 hours at room temperature. The reaction mixture was then filtered, and the filtrate was evaporated to give the crude product as an oil. This oil was triturated with a mixture of ethanol and water to give a solid which was recrystallized twice from a mixture of ethanol and water to give 5.3g of white crystalline N-(2-carboxyphenyl)-3,5-di-t-butyl-4-hydroxybenzamide, m.p. 202-203°C. Analysis: Calculated for $C_{22}H_{27}NO_4$: %C, 71.5; %H, 7.4; %N, 3.8; Found: %C, 71.4; %H, 7.5; %N, 3.7.

**EXAMPLE 4**

Preparation of 4-(3,5-Di-t-Butyl-4-Hydroxybenzamido)phenylacetic Acid

A suspension of 9.2g (0.061 mole) of p-aminophenylacetic acid in 150 ml of 1,2-dimethoxyethane was added to a suspension of 8.0g (0.0298 mole) of 3,5-di-t-butyl-4-hydroxybenzoyl chloride in 100 ml of 1,2-dimethoxyethane, and the resulting mixture was stirred at room temperature for 12 hours. The reaction mixture was then filtered. The filtrate was thereafter diluted with water and chilled. The resulting precipitate was collected and recrystallized from a mixture of ethanol and water to give 1.32g of yellow crystalline 4-(3,5-di-t-butyl-4-hydroxybenzamido)phenylacetic acid, m.p. 253-254°C. Analysis: Calculated for

$C_{23}H_{29}NO_4$: %C, 72.0; %H, 7.6; %N, 3.6; Found: %C, 72.0; %H, 7.7; %N, 3.4.

**EXAMPLE 5**

Preparation of N-(3-Carboxy-4-chlorophenyl)-3,5-di-t-butyl-4-hydroxybenzamide

A suspension of 6.90 g (0.04 mole) of 5-amino-2-chlorobenzoic acid in 100 ml of 1,2-dimethoxyethane was mixed with a solution of 5.08g (0.02 mole) of 3,5-di-t-butyl-4-hydroxybenzoyl chloride in 100 ml of 1,2-dimethoxyethane, and the mixture was stirred at 25° C. for about 72 hours. The reaction mixture was heated gently for 30 minutes and then allowed to cool before being filtered to remove the hydrochloride salt of the excess starting amine. The filtrate was concentrated under vacuum and then diluted with water. The resulting precipitate was collected, and was then recrystallized twice from a mixture of ethanol and water to give 2.8g of off-white solid N-(3-carboxy-4-chlorophenyl)-3,5-di-t-butyl-4-hydroxybenzamide, m.p. 265-267° C. Analysis: Calculated for $C_{22}H_{26}NO_4C1$: %C, 65.4; %H, 6.5; %N, 3.4; Found: %C, 65.8; %H, 6.6; %N, 3.5.

**EXAMPLE 6**

Preparation of N-(5-Carboxy-2-methoxyphenyl)-3,5-di-t-butyl-4-hydroxybenzamide

A solution of 5.06g (0.02 mole) of 3,5-di-t-butyl-4-hydroxybenzoyl chloride in 100 ml of 1,2-dimethoxyethane was mixed with a solution of 6.69g (0.04 mole) of 3-amino-4-methoxybenzoic acid in 125 ml of 1,2-dimethoxyethane, and the mixture was stirred at 25° C for about 16 hours. The reaction mixture was heated gently for 30 minutes and then allowed to cool before being filtered to remove the hydrochloride salt of the excess starting amine. The filtrate was concentrated under vacuum and then diluted with water. The resulting precipitate was collected and was then recrystallized three times from a mixture of ethanol and water to give 1.9g of white solid N-(5-carboxy-2-methoxyphenyl)-3,5-di-t-butyl-4-hydroxybenzamide, m.p. 259-261° C. Analysis: Calculated for $C_{23}H_{29}NO_5$: %C, 69.2; %H, 7.3; %N, 3.5; Found: %C, 69.3; %H, 7.3; %H, 3.6.

**EXAMPLE 7**

Preparation of 5-[3-(3,5-di-t-butyl-4-hydroxybenzamido)phenyl]tetrazole hydrate

Part A

Under a nitrogen atmosphere, a solution of 9.02 g (0.033 mole) of 3,5-di-t-butyl-4-hydroxybenzoyl chloride in 75 ml of 1,2-dimethoxyethane was mixed with a solution of 7.93g (0.067 mole) of 3-aminobenzonitrile in 75 ml of 1,2-dimethoxyethane. The resulting mixture was stirred at 25° C for an hour and then heated to a gentle reflux for about five hours. The mixture was allowed to cool and the resulting precipitate was collected and dried to give 10.3g of a tan crystalline solid. This material was then recrystallized from ethanol. Two different crops were obtained. The first gave 4.6g of an off-white crystalline material, m.p. 267-269° C. The second gave 2.4g of white crystalline N-(3-cyanophenyl)-3,5-di-t-butyl-4-hydroxybenzamide, m.p. 265-267° C. Analysis: Calculated for $C_{22}H_{26}N_2O_2$: %C, 75.4; %H, 7.5; %N, 8.0; Found: %C, 75.4; %H, 7.5; %N, 7.8.

Part B

A solution of 5.16g (0.015 mole) of N-(3-cyanophenyl)-3,5-di-t-butyl-4-hydroxybenzamide, 2.95g (0.045 mole) of sodium azide, 2.48g (0.045 mole) of ammonium chloride and 0.65g (0.015 mole) of lithium chloride in 30 ml of N,N-dimethylformamide was heated under a nitrogen atmosphere in a stoppered flask at about 120° C for about 60 hours. The reaction mixture was allowed to cool before being poured into a mixture of 10% hydrochloric acid and ice. The resulting precipitate was collected and then recrystallized from a mixture of ethanol and water to give 2.8g of off-white solid 5-[3-(3,5-di-t-butyl-4-hydroxybenzamido)phenyl]-tetrazole hydrate, m.p. 184-193° C. Analysis: Calculated for $C_{22}H_{27}N_5O_2 \cdot H_2O$: %C, 64.5; %H, 6.9; %N, 17.8; Found: %C, 64.9; %H, 7.0; %N, 17.4.

**EXAMPLE 8**

Preparation of 2-[N-(3,5-Di-t-butyl-4-hydroxyphenyl)carbamoyl]benzoic Acid

A solution of 5.60 g (0.03 mole) of the known compound 4-amino-2,6-di-t-butylphenol in 100 ml of diethyl ether was added dropwise over a period of twenty minutes to a solution of 4.44 g (0.03 mole) of phthalic anhydride in 200 ml of a 1:2 mixture of diethyl ether:toluene. The reaction mixture was stirred at room temperature for about sixteen hours. The resulting precipitate was collected, rinsed with a mixture of diethylether and hexane, and dried to give 6.8 g of a white solid. This material was recrystallized from a mixture of ethyl acetate and hexane to give 5.9 g of white solid 2-[N-(3,5-di-t-butyl-4-hydroxyphenyl)-carbamoyl]benzoic acid, m.p. 191-192 °C. Analysis: Calculated for $C_{22}H_{27}NO_4$: %C, 71.5; %H, 7.4, %N, 3.8; Found: %C, 71.8, %H, 7.3; %N, 3.7.

**EXAMPLE 9**

Preparation of 2-[N-(3,5-Di-t-butyl-4-hydroxyphenyl)carbamoyl]-cis-cyclohexanecarboxylic Acid

A solution containing 5.68 g (0.03 mole) of 4-amino-2,6-di-t-butylphenol in 200 ml of diethyl ether was added dropwise to a solution of 4.62 g (0.03 mole) of cis-1,2-cyclohexanedicarboxylic anhydride in 200 ml of diethyl ether. The reaction mixture was stirred at room temperature for about sixteen hours. The resulting precipitate was collected, rinsed with a mixture of diethyl ether and hexane, and dried to give 8.1 g of a solid. This material was recrystallized from a mixture of ethyl acetate and hexane to give 7.3 g of white solid 2-[N-(3,5-di-t-butyl-4-hydroxyphenyl)carbamoyl]-cis-cyclohexanecarboxylic acid, m.p. 181-184 °C. Analysis: Calculated for $C_{22}H_{33}NO_4$: %C, 70.4, %H, 8.9; %N, 3.7; Found: %C, 70.1; %H, 8.7; %N, 3.6.

**EXAMPLE 10**

Preparation of 2-[N-(3,5-Di-t-butyl-4-hydroxyphenyl)carbamoyl]-trans-cyclohexanecarboxylic Acid

A solution containing 4.00 g (0.022 mole) of 4-amino-2,6-di-t-butylphenol in 50 ml of 1,2-dimethoxyethane was added dropwise over a period of ten minutes to a solution of 3.35 g (0.022 mole) of trans-1,2-cyclohexanedicarboxylic anhydride in 50 ml of 1,2-dimethoxyethane. The reaction mixture was stirred at room temperature for about seventy-two hours. The solvent was removed under vacuum and the residual solid was triturated with a mixture of diethyl ether and hexane. The resulting solid was recrystallized from a mixture of ethyl acetate and hexane to give 4.4 g of white solid 2-[N-(3,5-di-t-butyl-4-hydroxyphenyl) carbamoyl]-trans-cyclohexanecarboxylic acid, m.p. 247-252 °C. Analysis: Calculated for $C_{22}H_{33}NO_4$: %C, 70.4; %H, 8.9; %N, 3.7; Found: %C, 70.4; %H, 8.9; %N, 3.6.

**EXAMPLE 11**

Preparation of 3-Carboxy-N-(3′,5′-di-t-butyl-4′-hydroxyphenyl)benzamide

To a suspension of 5.3 g (0.029 mole) of m-(chloroformyl)benzoic acid in a mixture of 5.8 g (0.058 mole) of triethyl amine and 100 ml of methylene chloride was added 6.4 g (0.029 mole) of 4-amino-2,6-di-t-butylphenol. The resulting solution was stirred at room temperature for about sixteen hours. The solvents were removed under vacuum and the residual solid was taken up in chloroform and washed with 3N hydrochloric acid. The chloroform solution was dried with magnesium sulfate and then evaporated to give 8 g of a solid. This material was slurried in 80 ml of 20% chloroform in hexane, collected, rinsed with chilled hexane and dried to give 5.9 g of a solid. This solid was slurried in 100 ml of warm 1:1 ethyl acetate:hexane, cooled, collected, rinsed with chilled hexane and dried to give 3.2g of pale pink solid. This pale pink solid was stirred in 320 ml refluxing chloroform. The hot chloroform was filtered to remove insoluble material. The filtrate was diluted with 500 ml of hexane and the resulting precipitate was collected and dried to give 0.97 g of a very pale pink solid. This solid was then slurried in 25% acetone in hexane, collected and dried to give 0.67 g of white solid 3-carboxy-N-(3′,5′di-t-butyl-4′-hydroxyphenyl)benzamide, m.p. 253-257 °C. Analysis: Calculated for $C_{22}H_{27}NO_4$: %C, 71.5; %H, 7.4; %N, 3.8; Found: %C, 71.9; %H, 7.4; %N, 3.6.

**EXAMPLE 12**

Preparation of 3-Cyano-N-(3′,5′-Di-t-butyl-4′-hydroxyphenyl)benzamide

To a solution containing 3.4 g (0.015 mole) of 4-amino-2,6-di-t-butylphenol and 2.3 g (0.015 mole) of 3-cyanobenzoic acid in 50 ml of tetrahydrofuran was added a solution containing 3.2 g (0.015 mole) of N,N′-dicyclohexylcarbodiimide in 10 ml of tetrahydrofuran. A precipitate formed immediately. The reaction mixture was allowed to stir at room temperature for about sixteen hours. The reaction mixture was filtered and the filtrate was concentrated under vacuum to give 5.7 g of a black solid. This material was triturated with 250 ml of 20% chloroform in hexane to give a tan solid. The tan solid was collected and then heated to reflux for about one hour in 100 ml of 20% chloroform in hexane. This mixture was filtered hot to provide 3.0 g of a rose solid. The rose solid was heated with 85 ml of 9:8 ethyl acetate:hexane and then filtered while still hot. The filtrate was cooled and the resulting precipitate was collected to provide 0.90g of solid 3-cyano-N-(3′,5′-di-t-butyl-4′-hydroxyphenyl) benzamide, m.p. 219-222°C. Analysis: Calculated for $C_{22}H_{26}N_2O_2$: %C, 75.4; %H, 7.5; %N, 8.0; Found: %C, 75.4; %H, 7.5, %N, 8.0.

**EXAMPLE 13**

Preparation of N-(3′,5′-Di-t-butyl-4′-hydroxyphenyl)-3-(5′-tetrazolyl)benzamide

A mixture containing 2.9 g (8.28 mmole) of 3-cyano-N-(3′,5′-di-t-butyl-4′-hydroxyphenyl)benzamide, 1.6g (24.8 mmole) of sodium azide, 1.3 g (24.8 mmole) of ammonium chloride, 0.35 g (8.28 mmole) of lithium chloride and 20 ml of dimethylformamide was heated under a nitrogen atmosphere at 90°C for about ninety-six hours. The reaction mixture was allowed to cool to room temperature and then filtered. The filtrate was added to a mixture of 50 ml 3N hydrochloric acid and ice to provide a gummy red solid. The solvents were decanted off and the solid was triturated with chloroform to provide 2.8 g of a pale pink solid. This material was stirred with 50 ml of 5% hexane in chloroform and then filtered to provide 2.2 g of a white solid m.p. 215-217°C. 1.7 g of this material was recrystallized from a mixture of ethanol and water to provide 1.2 g of white solid N-(3′-5′-di-t-butyl-4′-hydroxyphenyl)-3-(5′-tetrazolyl)benzamide. m.p. 238-242°C. Analysis: Calculated for: $C_{22}H_{27}H_5O_2$: %C, 67.15; %H, 6.9; %N, 17.8; Found: %C, 66.7; %H, 6.9; %N, 17.8.

**EXAMPLE 14**

Preparation of N-(3′,5′-Di-t-butyl-4′-hydroxyphenyl)-4-(5′-tetrazolyl)benzamide

Part A

Using the method of Example 12, 3.4 g of 4-amino-2,6-di-t-butylphenol was reacted with 2.3 g of 4-cyanobenzoic acid to provide 2.7 g of 4-cyano-N-(3′,5′-di-t-butyl-4′-hydroxyphenyl)benzamide.

Part B

Using the method of Example 13 the nitrile prepared in Part A was converted to the tetrazole to provide 1.2 g of pale yellow N-(3′,5′-di-t-butyl-4′-hydroxyphenyl)-4-(5′-tetrazolyl)benzamide m.p. 252-254°C. Analysis: Calculated for $C_{22}H_{27}N_5O_2$: %C, 67.15; %H, 6.9; %N, 17.8; Found: %C, 67.0; %H, 7.0; %N, 17.9.

**EXAMPLES 15-20**

Table I illustrates compounds of Formula VII which could be prepared via reaction Scheme A from 3,5-di-t-butyl-4-hydroxybenzoyl chloride and the indicated compounds of Formula VI using the general method of Examples 1-6.

## Table I

| Ex. No. | Compound of Formula VI | Product of Formula VII |
|---|---|---|
| 15 | 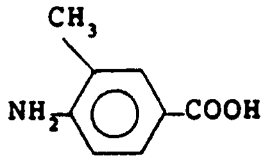 | 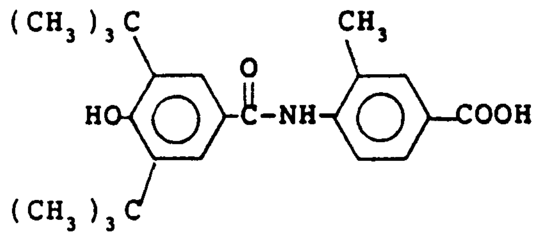 |
| 16 | 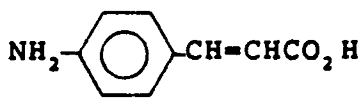 | 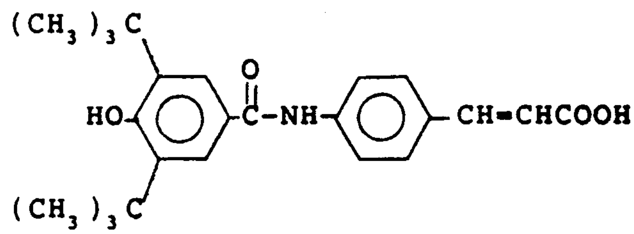 |
| 17 | 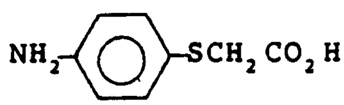 | 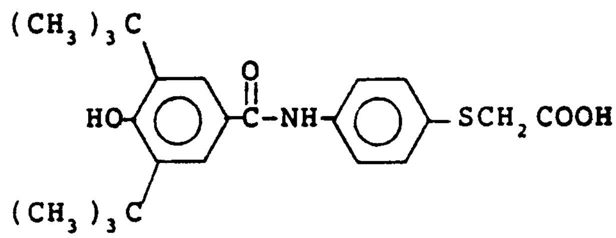 |
| 18 | 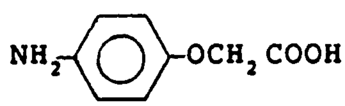 | 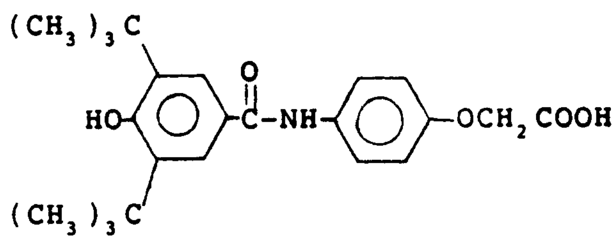 |
| 19 | 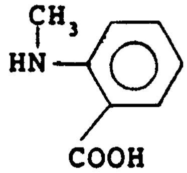 | 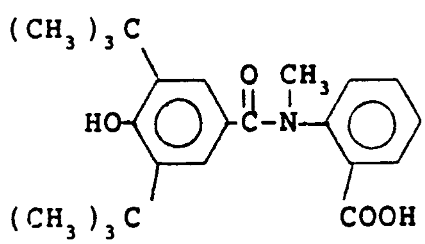 |

20 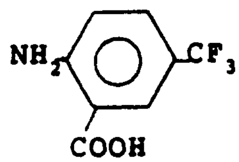 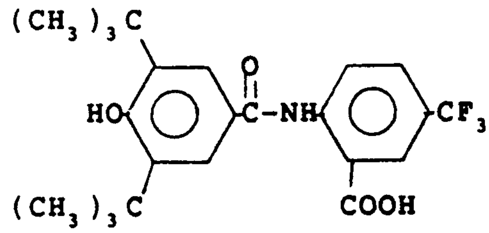

**EXAMPLES 21-27**

Table II illustrates compounds of Formula X which could be prepared via Reaction Scheme B from 3,5-di-t-butyl-4-hydroxybenzoyl chloride and the indicated compounds of Formula VIII via the indicated intermediates of Formula IX using the general method of Example 7.

## Table II

| Ex. No. | Compound of Formula VIII | Intermediate of Formula IX | Product of Formula X |
|---|---|---|---|
| 21 | 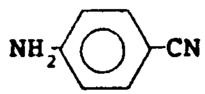 | 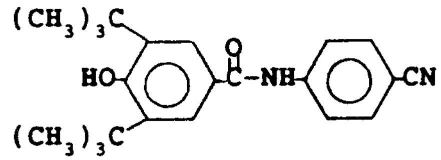 | 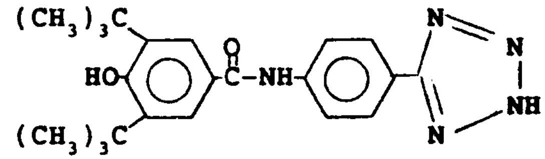 |
| 22 | 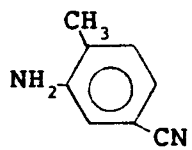 | 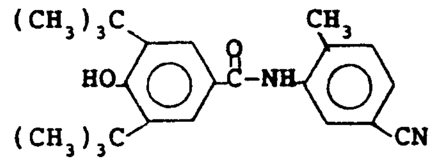 | 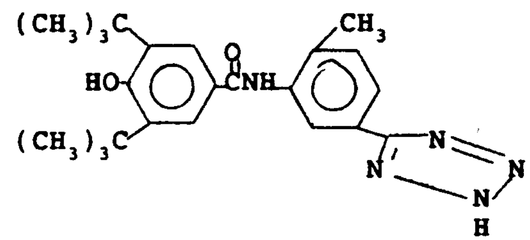 |
| 23 | 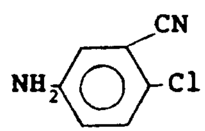 | 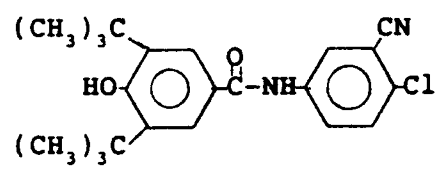 | 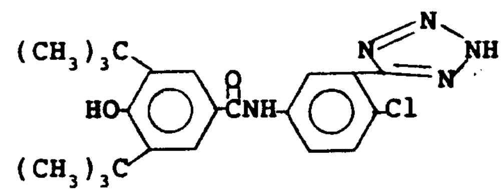 |
| 24 | 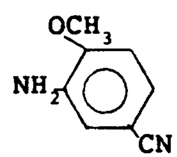 | 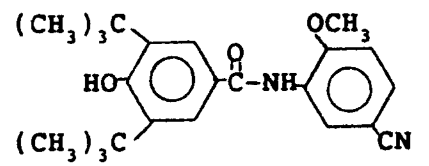 | 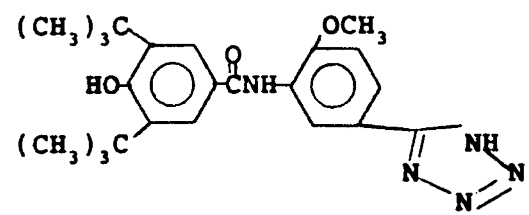 |

25 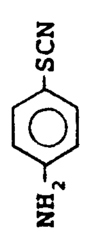 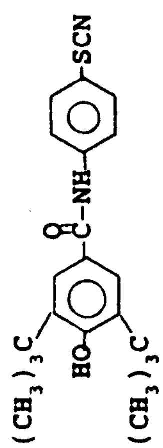 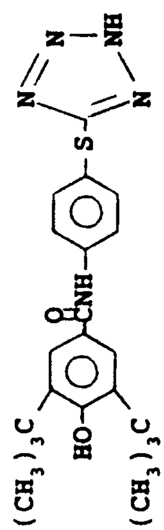

26 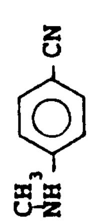 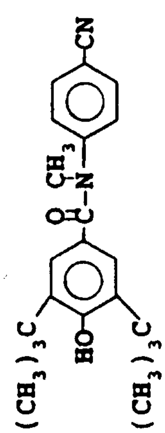 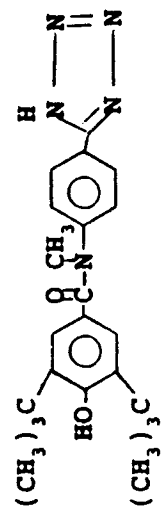

27 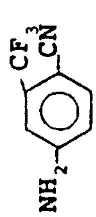 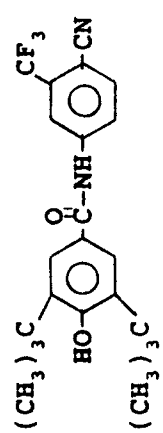 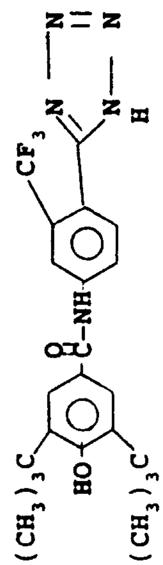

**EXAMPLES 28-32**

Table III illustrates compounds of Formula X which could be prepared via Reaction Scheme B from 3,5-di-t-butyl-4-hydroxybenzoyl chloride and the indicated compounds of Formula XI.

## Table III

| Ex. No. | Starting Material of Formula XI | Product of Formula X |
|---|---|---|
| 28 | 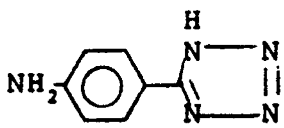 | 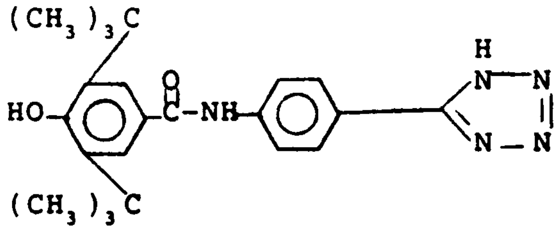 |
| 29 | 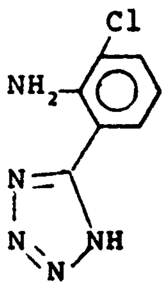 | 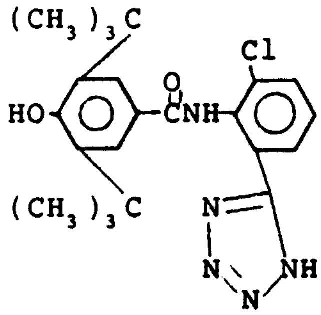 |
| 30 | 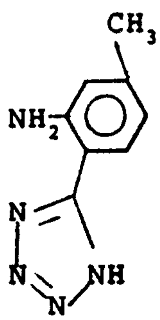 | 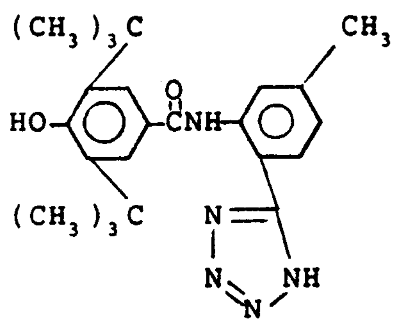 |

31 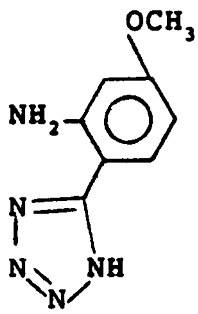 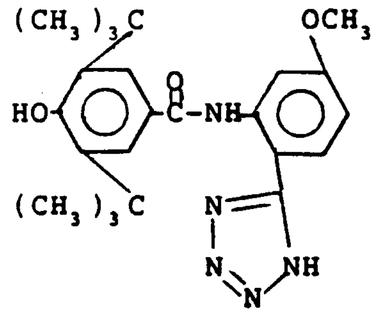

32 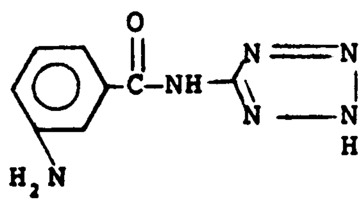 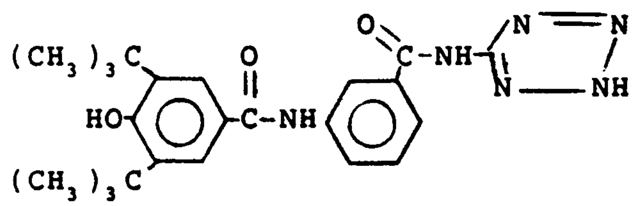

## Claims

### Claims for the following Contracting States : BE, CH, DE, FR, GB, IT, LI, NL, SE

**1.** A compound of the formula

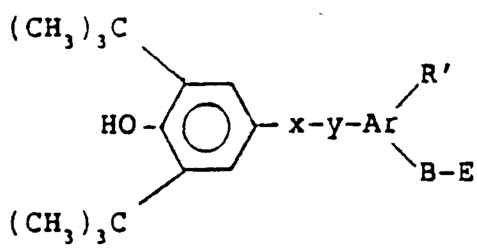

wherein x is carbonyl or N-R, and y is carbonyl or N-R; with the proviso that when x is carbonyl, y is N-R, and when x is N-R, y is carbonyl; further provided that:

1) when x is N-R,

R is hydrogen,

R' is hydrogen,

B is a carbon-carbon bond,

Ar is phenyl or cyclohexyl, and

E is tetrazolyl or carboxyl,

with the proviso that when Ar is cyclohexyl, E is carboxyl and is alpha to the carbonyl-cyclohexyl bond; and

2) when x is carbonyl,

R is hydrogen or lower alkyl,

R' is hydrogen, halogen, trifluoromethyl, lower alkyl or lower alkoxy,

Ar is phenyl,

E is carboxyl, tetrazolyl, or N-methyltetrazolyl, and

B is as follows:

when E is carboxyl, B is a carbon-carbon bond; an alkylene group containing one to seven carbon atoms; or an alkylene group containing one to seven carbon atoms and being either (a) interrupted by an ether linkage, a thioether linkage or a carbon-carbon double bond, or (b) attached to the phenyl ring by an oxygen atom or an unoxidized sulfur atom; or

when E is tetrazolyl or N-methyltetrazolyl, B is a carbon-carbon bond; an alkylene group containing

one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido;

or a derivative of said compound wherein E is carboxyl, selected from a lower alkyl ester, a (lower)-alkylamino(lower)alkyl ester, an ester of glycolamide, a pharmaceutically acceptable (lower)alkylamino-(lower)alkyl ester acid-addition salt and a pharmaceutically acceptable carboxylate salt;

or a derivative of said compound wherein E is tetrazolyl, selected from a pharmaceutically acceptable alkali metal or alkaline earth salt of the tetrazolyl moiety.

**2.** A compound according to Claim 1 wherein X is carbonyl

**3.** A compound according to Claim 1, wherein X is N-R.

**4.** A compound according to any one of claims 1 to 3, wherein Ar is cyclohexyl.

**5.** A compound according to any one of claims 1 to 3, wherein Ar is phenyl.

**6.** A compound according to any one of claims 1 to 5, wherein E is carboxyl.

**7.** A compound according to any one of claims 1 to 5, wherein E is tetrazolyl.

**8.** An antiallergic pharmaceutical composition comprising a compound according to Claim 1 and a pharmaceutically acceptable vehicle, the compound being present in an amount effective for obtaining an antiallergic response in a mammal.

**9.** A compound as defined in Claim 1 for use in a therapeutic treatment of the human or animal body.

**10.** A compound as defined in Claim 1 for use in a therapeutic treatment of the human or animal body comprising a treatment to inhibit bronchoconstriction due to an allergic response or to inhibit leukotriene biosynthesis.

**11.** Use of a compound as defined in Claim 1 in a process of preparing a medicament for inhibiting bronchoconstriction due to an allergic response in a mammal.

**12.** Use of a compound as defined in Claim 1 in a process of preparing a medicament for inhibiting leukotriene biosynthesis in a mammal.

**13.** A compound of the formula

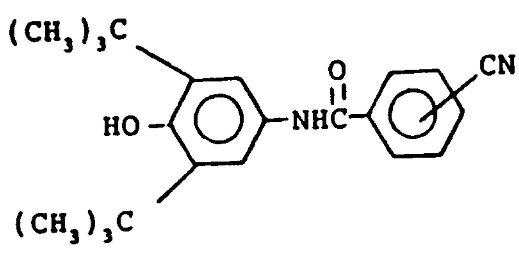

**14.** A compound of the formula

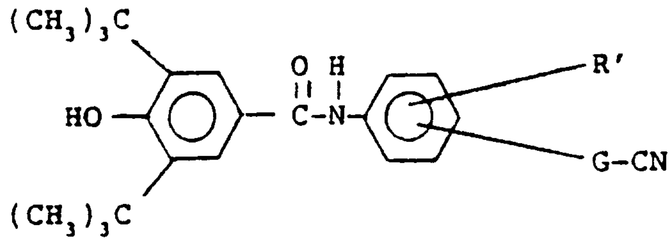

wherein

R' is hydrogen, halogen, trifluoromethyl, lower alkyl or lower alkoxy;

G is a carbon-carbon bond or alkylene of one to four carbon atoms.

**15.** A process for preparing a compound of the formula

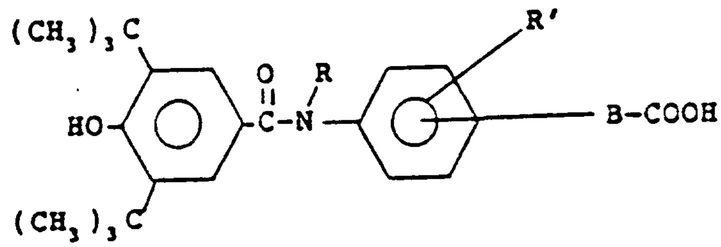

VII

comprising combining 3,5-di-t-butyl-4-hydroxybenzoyl chloride with an amine of the formula

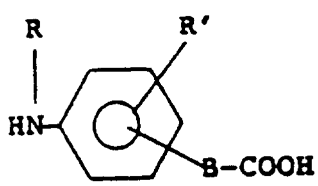

VI

in an inert solvent, accompanied by gentle heating if necessary; wherein R is hydrogen or lower alkyl; R' is hydrogen, halogen, trifluoremethyl, lower alkyl or lower alkoxy; and B is a carbon-carbon bond; an alkylene group containing one to seven carbon atoms, or an alkylene group containing one to seven carbon atoms and being either (a) interrupted by an ether linkage, a thioether linkage, or a carbon-carbon double bond, or (b) attached to the phenyl ring by an oxygen atom or an unoxidized sulfur atom.

**16.** A process for preparing a compound of the formula

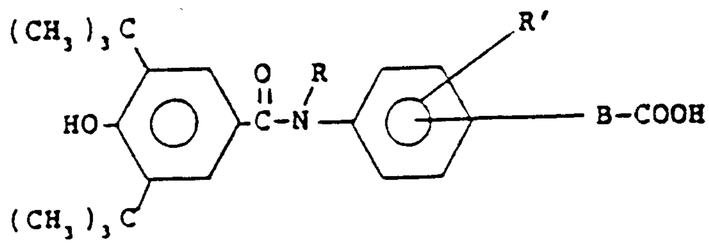

VII

comprising preferentially hydrolyzing the -CN group of a compound of the formula

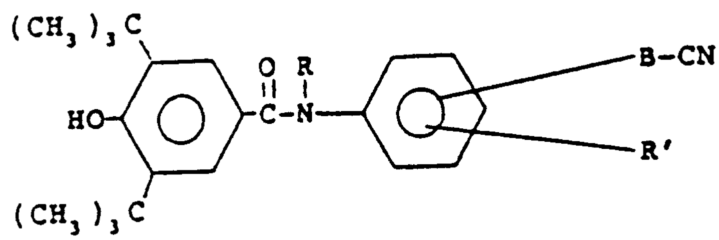

IX

wherein R is hydrogen or lower alkyl; R' is hydrogen, halogen, trifluoromethyl, lower alkyl or lower alkoxy; and B is a carbon-carbon bond; an alkylene group containing one to seven carbon atoms, or an alkylene group containing one to seven carbon atoms and being either (a) interrupted by an ether linkage, a thioether linkage, or a carbon-carbon double bond, or (b) attached to the phenyl ring by an oxygen atom or an unoxidized sulfur atom.

**17.** A process for preparing a compound of the formula

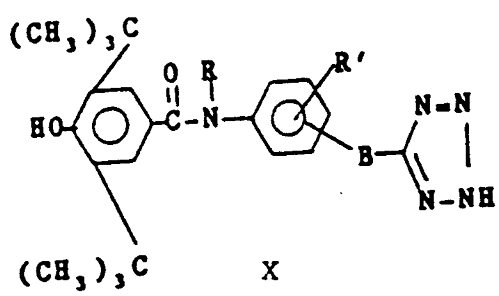

X

comprising combining a compound of the formula

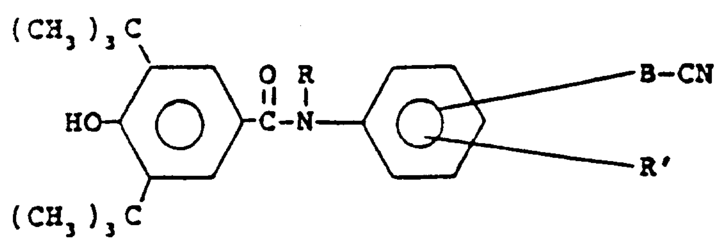

IX

with sodium azide, ammonium chloride and lithium chloride in the presence of a polar solvent, wherein R is hydrogen or lower alkyl; R' is hydrogen, halogen, trifluoromethyl, lower alkyl or lower alkoxy; and B is a carbon-carbon bond; an alkylene group containing one to four carbon atoms; an alkylene group

containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido.

**18.** A process for preparing a compound of the formula

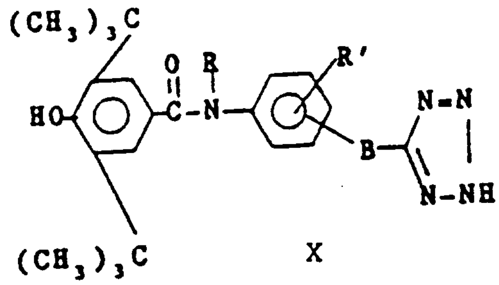

X

comprising combining 3,5-di-t-butyl-4-hydroxybenzoyl chloride and a compound of the formula

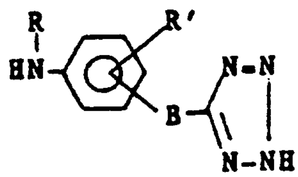

XI

in the presence of an inert solvent; wherein R is hydrogen or lower alkyl; R' is hydrogen, halogen, trifluoromethyl, lower alkyl or lower alkoxy; and B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido.

**Claims for the following Contracting State : ES**

**1.** A process for preparing a compound of the formula

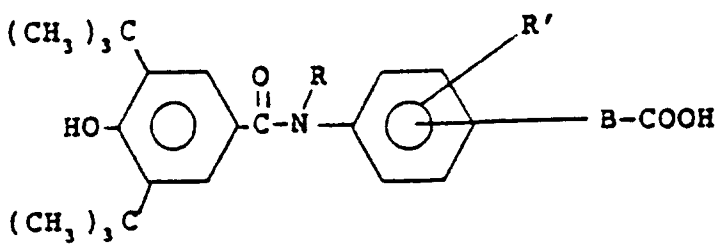

VII

comprising combining 3,5-di-t-butyl-4-hydroxybenzoyl chloride with an amine of the formula

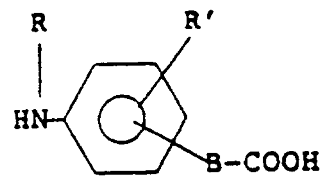

VI

in an inert solvent, accompanied by gentle heating if necessary; wherein R is hydrogen or lower alkyl; R' is hydrogen, halogen, trifluoromethyl, lower alkyl or lower alkoxy; and B is a carbon-carbon bond; an alkylene group containing one to seven carbon atoms, or an alkylene group containing one to seven carbon atoms and being either (a) interrupted by an ether linkage, a thioether linkage, or a carbon-carbon double bond, or (b) attached to the phenyl ring by an oxygen atom or an unoxidized sulfur atom.

**2.** A process for preparing a compound of the formula

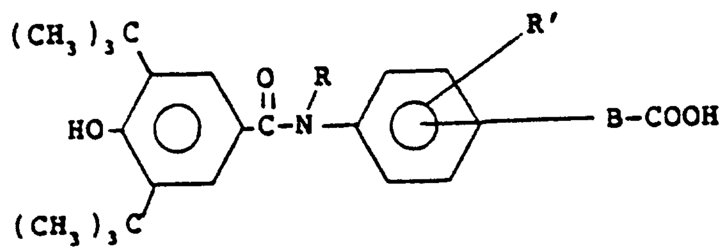

VII

comprising preferentially hydrolyzing the -CN group of a compound of the formula

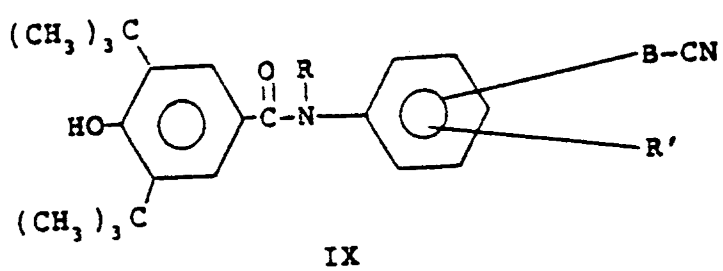

IX

wherein R is hydrogen or lower alkyl; R' is hydrogen, halogen, trifluoromethyl, lower alkyl or lower alkoxy; and B is a carbon-carbon bond; an alkylene group containing one to seven carbon atoms, or an alkylene group containing one to seven carbon atoms and being either (a) interrupted by an ether linkage, a thioether linkage, or a carbon-carbon double bond, or (b) attached to the phenyl ring by an oxygen atom or an unoxidized sulfur atom.

**3.** A process for preparing a compound of the formula

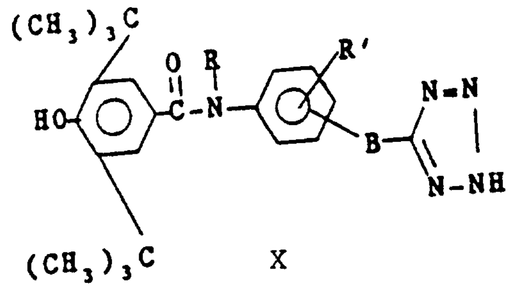

X

comprising combining a compound of the formula

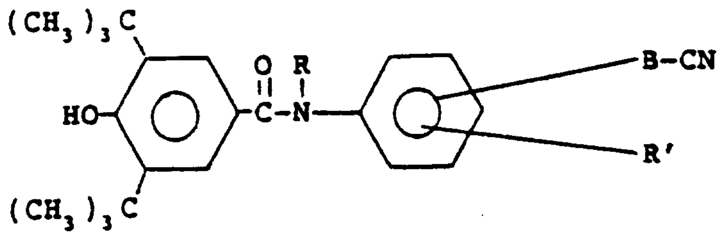

IX

with sodium azide, ammonium chloride and lithium chloride in the presence of a polar solvent, wherein R is hydrogen or lower alkyl; R' is hydrogen, halogen, trifluoromethyl, lower alkyl or lower alkoxy; and B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido.

**4.** A process for preparing a compound of the formula

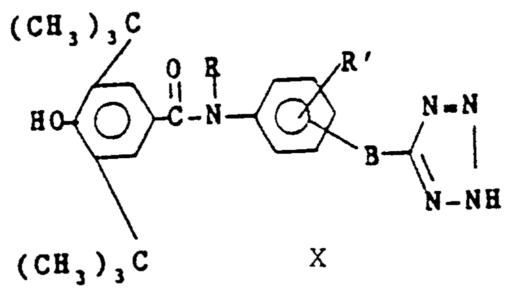

X

comprising combining 3,5-di-t-butyl-4-hydroxybenzoyl chloride and a compound of the formula

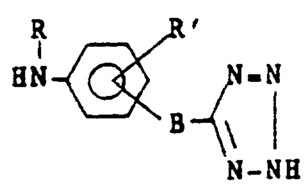

XI

in the presence of an inert solvent; wherein R is hydrogen or lower alkyl; R' is hydrogen, halogen, trifluoromethyl, lower alkyl or lower alkoxy; and B is a carbon-carbon bond; an alkylene group containing one to four carbon atoms; an alkylene group containing one to four carbon atoms and being

either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido.

**5.** Use of a compound of the formula

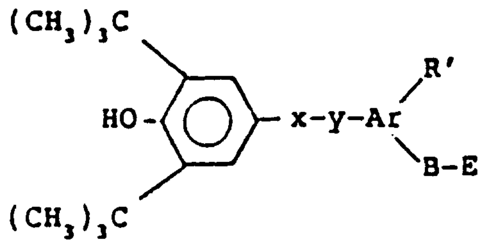

wherein x is carbonyl or N-R, and y is carbonyl or N-R; with the proviso that when x is carbonyl, y is N-R, and when x is N-R, y is carbonyl; further provided that:

1) when x is N-R,
R is hydrogen,
R' is hydrogen,
B is a carbon-carbon bond,
Ar is phenyl or cyclohexyl, and
E is tetrazolyl or carboxyl,
with the proviso that when Ar is cyclohexyl, E is carboxyl and is alpha to the carbonyl-cyclohexyl bond; and

2) when x is carbonyl,
R is hydrogen or lower alkyl,
R' is hydrogen, halogen, trifluoromethyl, lower alkyl or lower alkoxy,
Ar is phenyl,
E is carboxyl, tetrazolyl, or N-methyltetrazolyl, and
B is as follows:

when E is carboxyl, B is a carbon-carbon bond; an alkylene group containing one to seven carbon atoms; or an alkylene group containing one to seven carbon atoms and being either (a) interrupted by an ether linkage, a thioether linkage or a carbon-carbon double bond, or (b) attached to the phenyl ring by an oxygen atom or an unoxidized sulfur atom; or

when E is tetrazolyl or N-methyltetrazolyl, B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido;

or a derivative of said compound wherein E is carboxyl, selected from a lower alkyl ester, a (lower)-alkylamino(lower)alkyl ester, an ester of glycolamide, a pharmaceutically acceptable (lower)alkylamino-(lower)alkyl ester acid-addition salt and a pharmaceutically acceptable carboxylate salt;

or a derivative of said compound wherein E is tetrazolyl, selected from a pharmaceutically acceptable alkali metal or alkaline earth salt of the tetrazolyl moiety in a process for preparing a medicament for inhibiting bronchoconstriction due to an allergic response in a mammal.

**6.** Use of a compound of the formula

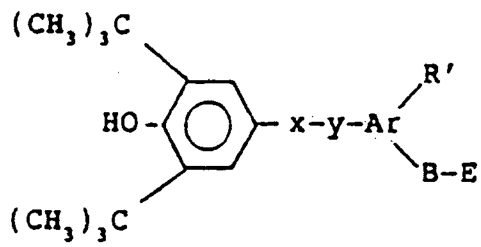

wherein x is carbonyl or N-R, and y is carbonyl or N-R; with the proviso that when x is carbonyl, y is N-R, and when x is N-R, y is carbonyl; further provided that:

1) when x is N-R,
R is hydrogen,
R' is hydrogen,
B is a carbon-carbon bond,
Ar is phenyl or cyclohexyl, and
E is tetrazolyl or carboxyl,
with the proviso that when Ar is cyclohexyl, E is carboxyl and is alpha to the carbonyl-cyclohexyl bond; and

2) when x is carbonyl,
R is hydrogen or lower alkyl,
R' is hydrogen, halogen, trifluoromethyl, lower alkyl or lower alkoxy,
Ar is phenyl,
E is carboxyl, tetrazolyl, or N-methyltetrazolyl, and
B is as follows:
when E is carboxyl, B is a carbon-carbon bond; an alkylene group containing one to seven carbon atoms; or an alkylene group containing one to seven carbon atoms and being either (a) interrupted by an ether linkage, a thioether linkage or a carbon-carbon double bond, or (b) attached to the phenyl ring by an oxygen atom or an unoxidized sulfur atom; or
when E is tetrazolyl or N-methyltetrazolyl, B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido;

or a derivative of said compound wherein E is carboxyl, selected from a lower alkyl ester, a (lower)-alkylamino(lower)alkyl ester, an ester of glycolamide, a pharmaceutically acceptable (lower)alkylamino-(lower)alkyl ester acid-addition salt and a pharmaceutically acceptable carboxylate salt;
or a derivative of said compound wherein E is tetrazolyl, selected from a pharmaceutically acceptable alkali metal or alkaline earth salt of the tetrazolyl moiety in a process for preparing a medicament for inhibiting leukotriene biosynthesis in a mammal.

**7.** Use of a compound of the formula

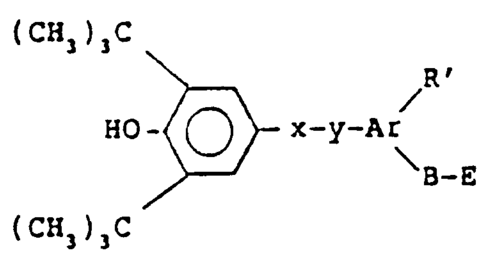

wherein x is carbonyl or N-R, and y is carbonyl or N-R; with the proviso that when x is carbonyl, y is N-R, and when x is N-R, y is carbonyl; further provided that:

1) when x is N-R,
R is hydrogen,
R' is hydrogen,
B is a carbon-carbon bond,
Ar is phenyl or cyclohexyl, and
E is tetrazolyl or carboxyl,
with the proviso that when Ar is cyclohexyl, E is carboxyl and is alpha to the carbonyl-cyclohexyl bond; and
2) when x is carbonyl,
R is hydrogen or lower alkyl,
R' is hydrogen, halogen, trifluoromethyl, lower alkyl or lower alkoxy,
Ar is phenyl,
E is carboxyl, tetrazolyl, or N-methyltetrazolyl, and
B is as follows:
when E is carboxyl, B is a carbon-carbon bond; an alkylene group containing one to seven carbon atoms; or an alkylene group containing one to seven carbon atoms and being either (a) interrupted by an ether linkage, a thioether linkage or a carbon-carbon double bond, or (b) attached to the phenyl ring by an oxygen atom or an unoxidized sulfur atom; or
when E is tetrazolyl or N-methyltetrazolyl, B is a carbon-carbon bond; an alkylene group containing one to four carbon atoms; an alkylene group containing one to four carbon atoms and being either (a) interrupted by an ether linkage, a thioether linkage or a carbon-carbon double bond, or (b) attached to the phenyl ring by an oxygen atom or an unoxidized sulfur atom; an unoxidized sulfur atom; or carboxamido;
or a derivative of said compound wherein E is carboxyl, selected from a lower alkyl ester, a (lower)-alkylamino(lower)alkyl ester, an ester of glycolamide, a pharmaceutically acceptable (lower)alkylamino-(lower)alkyl ester acid-addition salt and a pharmaceutically acceptable carboxylate salt;
or a derivative of said compound wherein E is tetrazolyl, selected from a pharmaceutically acceptable alkali metal or alkaline earth salt of the tetrazolyl moiety in a process of preparing a medicament for the therapeutic treatment of the human or animal body.

**Patentansprüche**
**Patentansprüche für folgende Vertragsstaaten : BE, CH, DE, FR, GB, IT, LI, NL, SE**

**1.** Verbindung der Formel

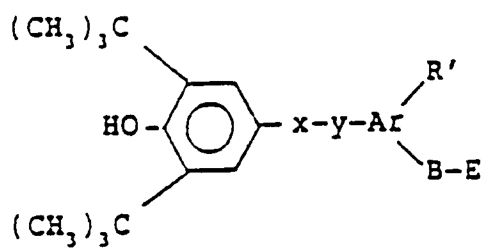

worin sind: x Carbonyl oder N-R und y Carbonyl oder N-R unter der Voraussetzung, daß, y N-R ist, wenn x Carbonyl ist, und y Carbonyl ist, wenn x N-R ist, sowie ferner unter der Voraussetzung, daß,
1) wenn x N-R ist, sind:
R Wasserstoff,
R' Wasserstoff,
B eine Kohlenstoff-Kohlenstoffbindung,
Ar Phenyl oder Cyclohexyl und
E Tetrazolyl oder Carboxyl,
mit der Bedingung, daß, wenn Ar Cyclohexyl ist, E Carboxyl ist und zu der Carbonyl-Cyclohexylbindung alpha ist; und
2) wenn x Carbonyl ist, sind:
R Wasserstoff oder niederes Alkyl,
R' Wasserstoff, Halogen, Trifluormethyl, niederes Alkyl oder niederes Alkoxy,

Ar Phenyl,
E Carboxyl, Tetrazolyl oder N-Methyltetrazolyl; und
für B gilt:

wenn E Carboxyl ist, ist B eine Kohlenstoff-Kohlenstoffbindung, eine Alkylen-Gruppe mit eins bis sieben Kohlenstoffatomen oder eine Alkylen-Gruppe mit eins bis sieben Kohlenstoffatomen und entweder (a) von einer Etherbindung, einer Tioetherbindung oder eine Kohlenstoff-Kohlenstoff-Doppelbindung unterbrochen oder (b) über ein Sauerstoffatom oder ein nichtoxidiertes Schwefelatom an dem Phenyl-Ring gebunden; oder

wenn E Tetrazolyl oder N-Methyltetrazolyl ist, ist B eine Kohlenstoff -Kohlenstoffbindung, eine Alkylen-Gruppe mit eins bis vier Kohlenstoffatomen, eine Alkylen-Gruppe mit eins bis vier Kohlenstoffatomen und entweder (a) von einer Etherbindung, einer Thioetherbindung oder einer Kohlenstoff-Kohlenstoff-Doppelbindung unterbrochen oder (b) an dem Phenyl-Ring gebunden über ein Sauerstoffatom oder ein nichtoxidiertes Schwefelatom, ein nichtoxidiertes Schwefelatom, oder Carboxamido;

oder ein Derivat der Verbindung, bei welcher E Carboxyl ist, ausgewählt aus niederem Alkylester, (niederem)Alkylamino(niederem)Alkylester, einem Ester des Glycolamids, einem pharmazeutisch zulässigen Säureanlagerungsalz des (niederen)Alkylamino(niederen)Alkylesters und einem pharmazeutisch zulässigen Carboxylatsalz;

oder ein Derivat der Verbindung, bei welcher E Tetrazolyl ist, ausgewählt aus einem phamazeutisch zulässigen Alkalimetall- und Erdalkalimetallsalz der Tetrazolyl-Gruppe.

**2.** Verbindung nach Anspruch 1, bei welcher X Carbonyl ist.

**3.** Verbindung nach Anspruch 1, bei welcher Y N-R ist.

**4.** Verbindung nach Anspruch 1 bis 3, bei welcher Ar Cyclohexyl ist.

**5.** Verbindung nach Anspruch 1 bis 3, bei welcher Ar Phenyl ist.

**6.** Verbindung nach Anspruch 1 bis 5, bei welcher E Carboxyl ist.

**7.** Verbindung nach Anspruch 1 bis 5, bei welcher E Tratazolyl ist.

**8.** Antiallergische phamazeutische Zusammensetzung, umfassend eine Verbindung nach Anspruch 1 und einen pharmazeutisch zulässigen Träger, wobei die Verbindung in einer wirksamen Menge vorliegt, um in einem Säuger eine antiallergische Reaktion zu erzielen.

**9.** Verbindung, wie unter Anspruch 1 festgelegt, zur Verwendung in einer therapeutischen Behandlung des Menschen oder Tieres.

**10.** Verbindung, wie unter Anspruch 1 festgelegt, zur Verwendung in einer therapeutischen Behandlung des Menschen oder Tieres, umfassend eine Behandlung zum Hemmen von Bronchokonstriktionen als Folge einer allergischen Reaktion oder zum Hemmen von Leukotrien-Biosynthese.

**11.** Verwendung einer Verbindung, wie unter Anspruch 1 festgelegt, in einem Verfahren zur Herstellung eines Medikaments zum Hemmen von Bronchokonstriktionen als Folge einer allergischen Reaktion in einem Säuger.

**12.** Verwendung einer Verbindung, wie unter Anspruch 1 festgelegt, in einem Verfahren zur Herstellung eines Medikaments zum Hemmen von Leukotrien-Biosynthese in einem Säuger.

**13.** Verbindung der Formel

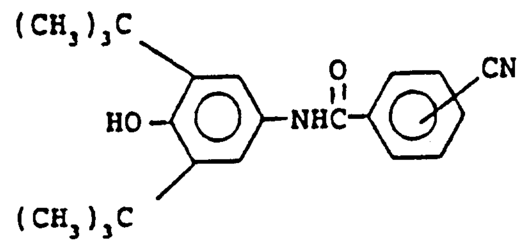

**14.** Verbindung der Formel

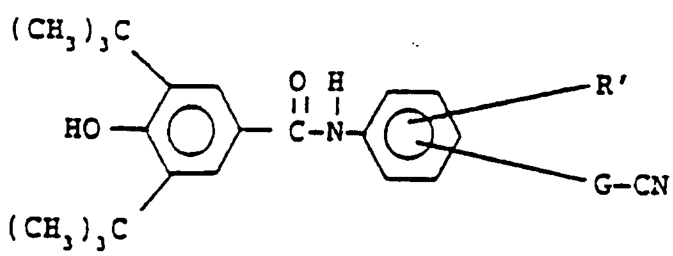

worin sind:

R' Wasserstoff, Halogen, Trifluormethyl, niederes Alkyl oder niederes Alkoxy;

G eine Kohlenstoff-Kohlenstoffbindung oder Alkylen mit eins bis vier Kohlenstoffatomen.

**15.** Verfahren zur Herstellung einer Verbindung der Formel

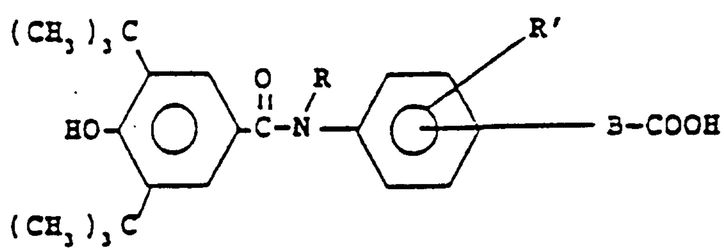

VII

umfassend das Vereinen von 3,5-Di-*tert*-butyl-4-hydroxybenzoylchlorid mit einem Amin der Formel

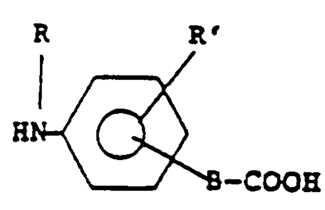

VI

in einem inerten Lösemittel, wenn erforderlich unter leichtem Erhitzen, worin R Wasserstoff oder niederes Alkyl ist, R' Wasserstoff ist, Halogen, Trifluormethyl, niederes Alkyl oder niederes Alkoxy und B eine Kohlenstoff-Kohlenstoffbindung ist, eine Alkylen-Gruppe mit einem bis sieben Kohlenstoffatomen oder eine Alkylen-Gruppe mit einem bis sieben Kohlenstoffatomen und entweder (a) von einer

Etherbindung, einer Thioetherbindung oder einer Kohlenstoff-Kohlenstoff-Doppelbindung unterbrochen oder (b) am Phenyl-Ring über ein Sauerstoffatom oder ein nichtoxidiertes Schwefelatom gebunden.

**16.** Verfahren zur Herstellung einer Verbindung der Formel

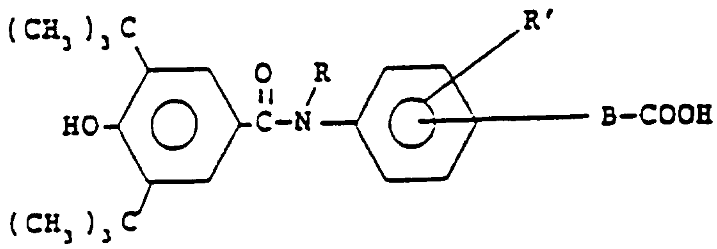

VII

umfassend vorzugsweise Hydrolysieren der -CN-Gruppe einer Verbindung der Formel

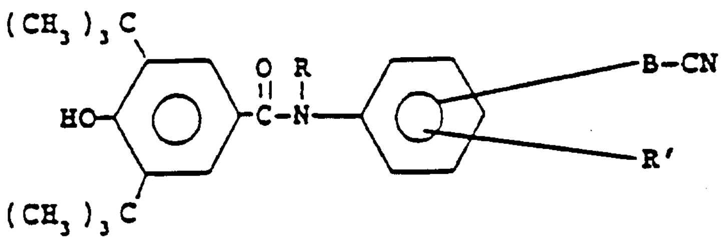

IX

worin R Wasserstoff oder niederes Alkyl ist; R' Wasserstoff, Halogen, Trifluormethyl, niederes Alkyl oder niederes Alkoxy ist; und B eine Kohlenstoff-Kohlenstoffbindung ist, eine Alkylen-Gruppe mit einem bis sieben Kohlenstoffatomen oder eine Alkylen-Gruppe mit einem bis sieben Kohlenstoffatomen und entweder (a) von einer Etherbindung, einer Thioetherbindung oder einer Kohlenstoff-Kohlenstoff-Doppelbindung unterbrochen oder (b) über ein Sauerstoffatom oder nichtoxidiertes Schwefelatom an dem Phenyl-Ring gebunden.

**17.** Verfahren zur Herstellung einer Verbindung der Formel

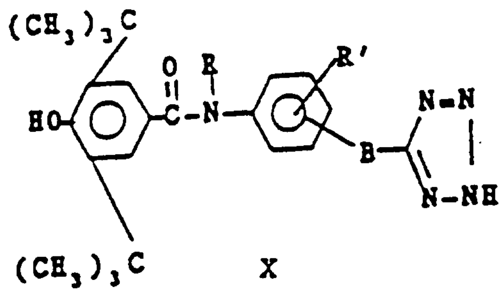

X

umfassend das Vereinen einer Verbindung der Formel

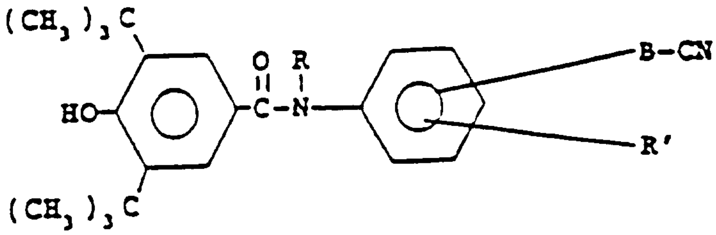

IX

mit Natriumazid, Ammoniumchlorid und Lithiumchlorid in Gegenwart eines polaren Lösemittels, worin R Wasserstoff oder niederes Alkyl ist, R' Wasserstoff, Halogen, Trifluormethyl, niederes Alkyl oder niederes Alkoxy ist und B eine Kohlenstoff-Kohlenstoffbindung, eine Alkylen-Gruppe mit einem bis vier Kohlenstoffatomen, eine Alkylen-Gruppe mit einen bis vier Kohlenstoffatomen und entweder (a) von einer Etherbindung, einer Thioetherbindung einer Kohlenstoff-Kohlenstoff-Doppelbindung unterbrochen oder (b) an dem Phenyl-Ring gebunden über ein Sauerstoffatom oder ein nichtoxidiertes Schwefelatom, ein nichtoxidiertes Schwefelatom, oder Carboxamido.

**18.** Verfahren zur Herstellung einer Verbindung der Formel

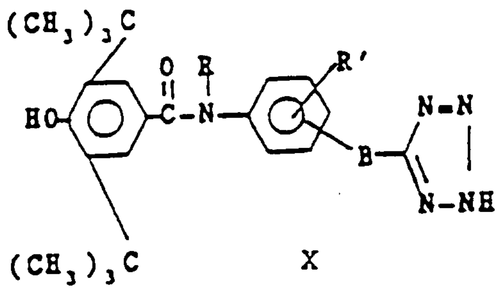

X

umfassend das Vereinen von 3,5-Di-*tert*-butyl-4-hydroxybenzoylchlorid und einer Verbindung der Formel

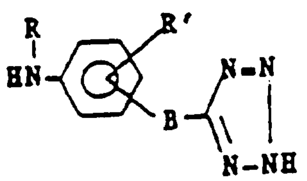

XI

in Gegenwart eines inerten Lösemittels, worin R Wasserstoff oder niederes Alkyl ist, R' Wasserstoff, Halogen, Trifluormethyl, niederes Alkyl oder niederes Alkoxy ist und B eine Kohlenstoff-Kohlenstoffbindung, eine Alkylen-Gruppe mit einem bis vier Kohlenstoffatomen, eine Alkylen-Gruppe mit eine bis vier Kohlenstoffatomen und entweder (a) von einer Etherbindung, einer Thioetherbindung oder einer Kohlenstoff-Kohlenstoff-Doppelbindung unterbrochen oder (b) an dem Phenyl-Ring gebunden über ein Sauerstoffatom oder ein nichtoxidiertes Schwefelatom, ein nichtoxidiertes Schwefelatom, oder Carboxamido.

**Patentansprüche für folgenden Vertragsstaat : ES**

1. Verfahren zur Herstellung einer Verbindung der Formel

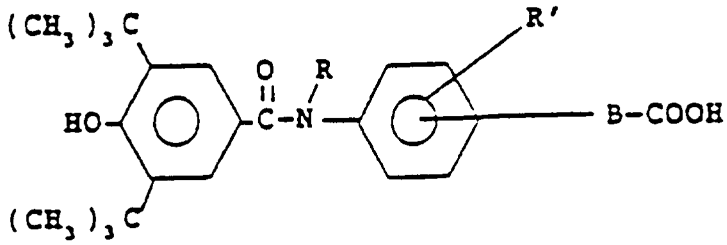

VII

umfassend das Vereinen von 3,5-Di-*tert*-butyl-4-hydroxybenzoylchlorid mit einem Amin der Formel

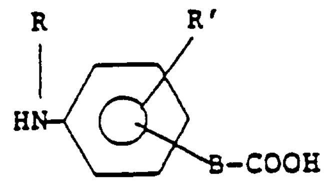

VI

in einem inerten Lösemittel, erforderlichenfalls unter leichtem Erhitzen, worin R Wasserstoff oder niederes Alkyl ist, R' Wasserstoff, Halogen, Trifluormethyl, niederes Alkyl oder niederes Alkoxy ist und B eine Kohlenstoff-Kohlenstoffbindung ist, eine Alkylen-Gruppe mit einem bis sieben Kohlenstoffatomen, oder eine Alkylen-Gruppe mit einem bis sieben Kohlenstoffatomen und entweder (a) von einer Etherbindung, einer Thioetherbindung oder einer Kohlenstoff-Kohlenstoff-Doppelbindung unterbrochen oder (b) an dem Phenyl-Ring gebunden über ein Sauerstoffatom oder ein nichtoxidiertes Schwefelatom.

2. Verfahren zur Herstellung einer Verbindung der Formel

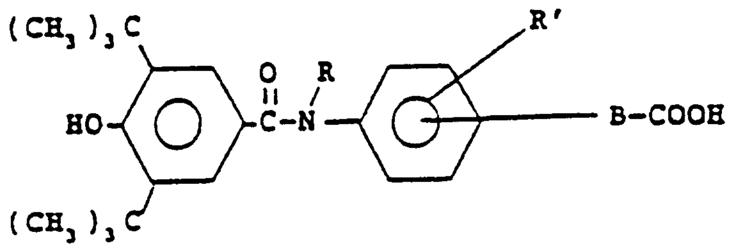

VII

umfassend vorzugsweise das Hydrolysieren der -CN-Gruppe einer Verbindung der Formel

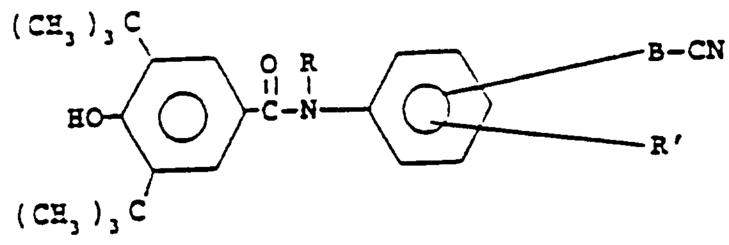

IX

worin R Wasserstoff oder niederes Alkyl ist, R' Wasserstoff, Halogen, Trifluormethyl, niederes Alkyl oder niederes Alkoxy ist und B eine Kohlenstoff-Kohlenstoffbindung, eine Alkylen-Gruppe mit einem bis sieben Kohlenstoffatomen oder eine Alkylen-Gruppe mit einem bis sieben Kohlenstoffatomen und entweder (a) von einer Etherbindung, einer Thioetherbindung oder einer Kohlenstoff-Kohlenstoff-Doppelbindung unterbrochen oder (b) über ein Sauerstoffatom oder ein nichtoxidiertes Schwefelatom an dem Phenyl-Ring gebunden.

**3.** Verfahren zur Herstellung einer Verbindung der Formel

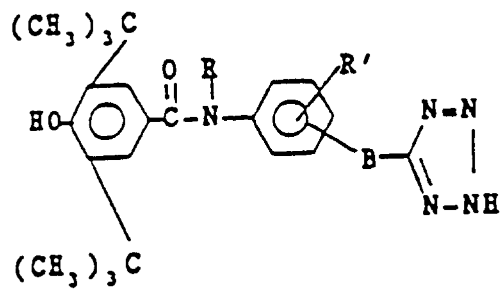

X

umfassend das Vereinen einer Verbindung der Formel

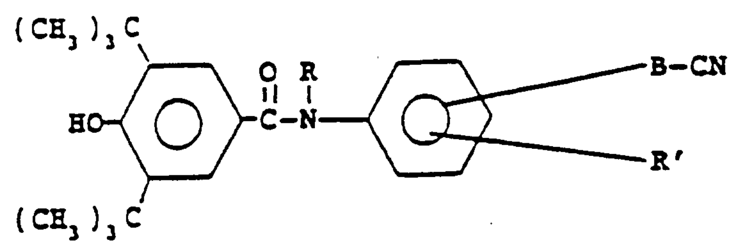

IX

mit Natriumazid, Ammoniumchlorid und Lithiumchlorid in Gegenwart eines polaren Lösemittels, worin R Wasserstoff oder niederes Alkyl ist, R' Wasserstoff, Halogen, Trifluormethyl, niederes Alkyl oder niederes Alkoxy ist und B eine Kohlenstoff-Kohlenstoffbindung ist, eine Alkylen-Gruppe mit einen bis vier Kohlenstoffatomen, eine Alkylen-Gruppe mit einem bis vier Kohlenstoffatomen und entweder (a) von einer Etherbindung, einer Thioetherbindung oder einen Kohlenstoff-Kohlenstoff-Doppelbindung unterbrochen oder (b) über ein Sauerstoffatom oder nichtoxidiertes Schwefelatom, ein nichtoxidiertes Schwefelatom, oder Carboxamido an dem Phenyl-Ring gebunden.

**4.** Verfahren zur Herstellung einer Verbindung der Formel

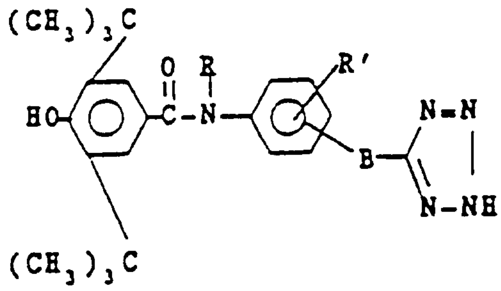

X

umfassend das Vereinen von 3,5-Di-*tert*-butyl-4-hydroxybenzoylchlorid und einer Verbindung der Formel

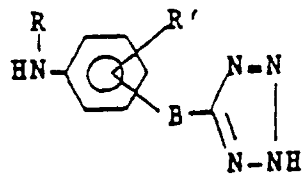

XI

in Gegenwart eines inerten Lösemittels, worin R Wasserstoff oder niederes Alkyl ist,

**5.** Verwendung einer Verbindung der Formel

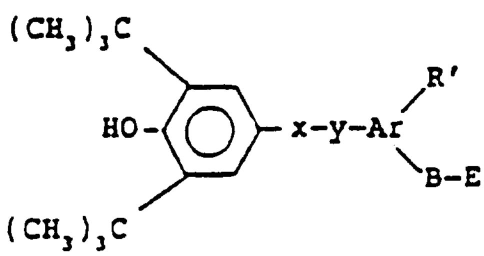

worin sind: x Carbonyl oder N-R und y Carbonyl oder N-R unter der Voraussetzung, daß, y N-R ist, wenn x Carbonyl ist, und y Carbonyl ist, wenn x N-R ist, sowie ferner unter der Voraussetzung, daß,

1) wenn x N-R ist, sind:

R Wasserstoff,
R' Wasserstoff,
B eine Kohlenstoff-Kohlenstoffbindung,
Ar Phenyl oder Cyclohexyl und
E Tetrazolyl oder Carboxyl,

mit der Bedingung, daß, wenn Ar Cyclohexyl ist, E Carboxyl ist und zu der Carbonyl-Cyclohexylbindung alpha ist; und

2) wenn x Carbonyl ist, sind:

R Wasserstoff oder niederes Alkyl,
R' Wasserstoff, Halogen, Trifluormethyl, niederes Alkyl oder niederes Alkoxy,

Ar Phenyl,
E Carboxyl, Tetrazolyl oder N-Methyltetrazolyl; und
für B gilt:
wenn E Carboxyl ist, ist B eine Kohlenstoff-Kohlenstoffbindung, eine Alkylen-Gruppe mit eins bis sieben Kohlenstoffatomen oder eine Alkylen-Gruppe mit eins bis sieben Kohlenstoffatomen und entweder (a) durch eine Etherbindung, eine Tioetherbindung oder eine Kohlenstoff-Kohlenstoff-Doppelbindung unterbrochen oder (b) über ein Sauerstoffatom oder ein nichtoxidiertes Schwefelatom an dem Phenyl-Ring gebunden; oder
wenn E Tetrazolyl oder N-Methyltetrazolyl ist, ist B eine Kohlenstoff-Kohlenstoffbindung, eine Alkylen-Gruppe mit eins bis vier Kohlenstoffatomen, eine Alkylen-Gruppe mit eins bis vier Kohlenstoffatomen und entweder (a) unterbrochen von einer Etherbindung, einer Thioetherbindung oder einer Kohlenstoff-Kohlenstoff-Doppelbindung, oder (b) an dem Phenyl-Ring gebunden über ein Sauerstoffatom oder ein nichtoxidiertes Schwefelatom, ein nichtoxidiertes Schwefelatom, oder Carboxamido;
oder ein Derivat der Verbindung, bei welcher E Carboxyl ist, ausgewählt aus niederem Alkylester, (niederem)Alkylamino(niederem)Alkylester, einem Ester des Glycolamids, einem pharmazeutisch zulässigen Säureanlagerungsalz des (niederen) Alkylamino (niederen) Alkylesters und einem pharmazeutisch zulässigen Carboxylatsalz;
oder ein Derivat der Verbindung, bei welcher E Tetrazolyl ist, ausgewählt aus einem phamazeutisch zulässigen Alkalimetall- und Erdalkalimetallsalz der Tetrazolyl-Gruppe
in einem Verfahren zur Herstellung eines Medikaments zum Hemmen von Bronchokonstriktion infolge einer allergischen Reaktion in einem Säuger.

**6.** Verwendung einer Verbindung der Formel

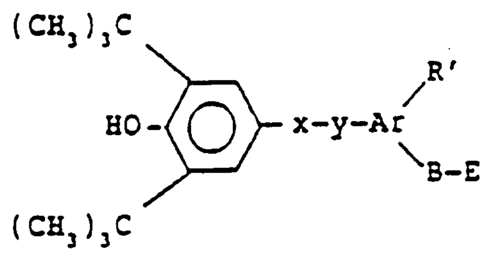

worin sind: x Carbonyl oder N-R und y Carbonyl oder N-R unter der Voraussetzung, daß, y N-R ist, wenn x Carbonyl ist, und y Carbonyl ist, wenn x N-R ist, sowie ferner unter der Voraussetzung, daß,
1) wenn x N-R ist, sind:
R Wasserstoff,
R' Wasserstoff,
B eine Kohlenstoff-Kohlenstoffbindung,
Ar Phenyl oder Cyclohexyl und
E Tetrazolyl oder Carboxyl,
mit der Bedingung, daß, wenn Ar Cyclohexyl ist, E Carboxyl ist und zu der Carbonyl-Cyclohexylbindung alpha ist; und
2) wenn x Carbonyl ist, sind:
R Wasserstoff oder niederes Alkyl,
R' Wasserstoff, Halogen, Trifluormethyl, niederes Alkyl oder niederes Alkoxy,
Ar Phenyl,
E Carboxyl, Tetrazolyl oder N-Methyltetrazolyl; und
für B gilt:
wenn E Carboxyl ist, ist B eine Kohlenstoff-Kohlenstoffbindung, eine Alkylen-Gruppe mit eins bis sieben Kohlenstoffatomen oder eine Alkylen-Gruppe mit eins bis sieben Kohlenstoffatomen und entweder (a) durch eine Etherbindung, eine Tioetherbindung oder eine Kohlenstoff-Kohlenstoff-Doppelbindung unterbrochen oder (b) über ein Sauerstoffatom oder ein nichtoxidiertes Schwefelatom an dem Phenyl-Ring gebunden; oder
wenn E Tetrazolyl oder N-Methyltetrazolyl ist, ist B eine Kohlenstoff-Kohlenstoffbindung, eine Alkylen-Gruppe mit eins bis vier Kohlenstoffatomen, eine Alkylen-Gruppe mit eins bis vier Kohlenstoffatomen und entweder (a) unterbrochen von einer Etherbindung, einer Thioetherbindung oder einer Kohlenstoff-Kohlenstoff-Doppelbindung, oder (b) an dem Phenyl-Ring gebunden über ein Sauerstoffatom oder ein nichtoxidiertes Schwefelatom, ein nichtoxidiertes Schwefelatom, oder Carboxamido;

oder ein Derivat der Verbindung, bei welcher E Carboxyl ist, ausgewählt aus niederem Alkylester, (niederem)Alkylamino(niederem)Alkylester, einem Ester des Glycolamids, einem pharmazeutisch zulässigen Säureanlagerungsalz des (niederen)Alkylamino(niederen)Alkylesters und einem pharmazeutisch zulässigen Carboxylatsalz;

oder ein Derivat der Verbindung, bei welcher E Tetrazolyl ist, ausgewählt aus einem phamazeutisch zulässigen Alkalimetall- und Erdalkalimetallsalz der Tetrazolyl-Gruppe

in einem Verfahren zur Herstellung eines Medikaments zum Hemmen von Leukotrien-Biosynthese in einem Säuger.

**7.** Verwendung einer Verbindung der Formel

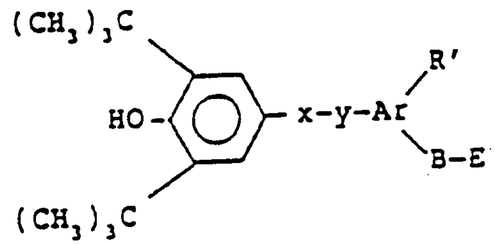

worin sind: x Carbonyl oder N-R und y Carbonyl oder N-R unter der Voraussetzung, daß, y N-R ist, wenn x Carbonyl ist, und y Carbonyl ist, wenn x N-R ist, sowie ferner unter der Voraussetzung, daß,

1) wenn x N-R ist, sind:

R Wasserstoff,
R' Wasserstoff,
B eine Kohlenstoff-Kohlenstoffbindung,
Ar Phenyl oder Cyclohexyl und
E Tetrazolyl oder Carboxyl,

mit der Bedingung, daß, wenn Ar Cyclohexyl ist, E Carboxyl ist und zu der Carbonyl-Cyclohexylbindung alpha ist; und

2) wenn x Carbonyl ist, sind:

R Wasserstoff oder niederes Alkyl,
R' Wasserstoff, Halogen, Trifluormethyl, niederes Alkyl oder niederes Alkoxy,
Ar Phenyl,
E Carboxyl, Tetrazolyl oder N-Methyltetrazolyl; und

für B gilt:

wenn E Carboxyl ist, ist B eine Kohlenstoff-Kohlenstoffbindung, eine Alkylen-Gruppe mit eins bis sieben Kohlenstoffatomen oder eine Alkylen-Gruppe mit eins bis sieben Kohlenstoffatomen und entweder (a) durch eine Etherbindung, eine Tioetherbindung oder eine Kohlenstoff-Kohlenstoff-Doppelbindung unterbrochen oder (b) über ein Sauerstoffatom oder ein nichtoxidiertes Schwefelatom an dem Phenyl-Ring gebunden; oder

wenn E Tetrazolyl oder N-Methyltetrazolyl ist, ist B eine Kohlenstoff-Kohlenstoffbindung, eine Alkylen-Gruppe mit eins bis vier Kohlenstoffatomen, eine Alkylen-Gruppe mit eins bis vier Kohlenstoffatomen und entweder (a) unterbrochen von einer Etherbindung, einer Thioetherbindung oder einer Kohlenstoff-Kohlenstoff-Doppelbindung, oder (b) an dem Phenyl-Ring gebunden über ein Sauerstoffatom oder ein nichtoxidiertes Schwefelatom, ein nichtoxidiertes Schwefelatom, oder Carboxamido;

oder ein Derivat der Verbindung, bei welcher E Carboxyl ist, ausgewählt aus niederem Alkylester, (niederem)Alkylamino(niederem)Alkylester, einem Ester des Glycolamids, einem pharmazeutisch zulässigen Säureanlagerungsalz des (niederen)Alkylamino(niederen)Alkylesters und einem pharmazeutisch zulässigen Carboxylatsalz;

oder ein Derivat der Verbindung, bei welcher E Tetrazolyl ist, ausgewählt aus einem phamazeutisch zulässigen Alkalimetall- und Erdalkalimetallsalz der Tetrazolyl-Gruppe

in einem Verfahren zur Herstellung eines Medikaments zum Hemmen zur therapeutuschen Behandlung des Körpers von Mensch und Tier.

**Revendications**

**Revendications pour les Etats contractants suivants : BE, CH, DE, FR, GB, IT, LI, NL, SE**

**1.** Composé de formule

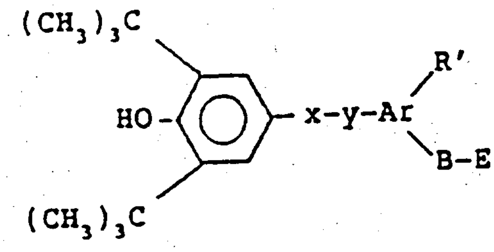

dans laquelle x représente un groupe carbonyle ou N-R, et y représente un groupe carbonyle ou N-R ; sous réserve que, lorsque x représente un groupe carbonyle, y représente un groupe N-R et, lorsque x représente un groupe N-R, y représente un groupe carbonyle ; sous réserve en outre que :

1) lorsque x représente un groupe N-R,

- R représente l'hydrogène,
- R' représente l'hydrogène,
- B représente une liaison carbone-carbone,
- Ar représente un groupe phényle ou cyclohexyle et
- E représente un groupe tétrazolyle ou carboxyle,

sous réserve que, lorsque Ar représente un groupe cyclohexyle, E représente un groupe carboxyle et soit en position alpha par rapport à la liaison carbonyle-cyclohexyle ; et

2) lorsque x représente un groupe carbonyle,

- R représente l'hydrogène ou un groupe alkyle inférieur,
- R' représente l'hydrogène, un halogène, un groupe trifluorométhyle, alkyle inférieur ou alkoxy inférieur,
- Ar représente un groupe phényle,
- E représente un groupe carboxyle, tétrazolyle ou N-méthyltétrazolyle, et
- B réponde à la définition suivante :

lorsque E représente un groupe carboxyle, B représente une liaison carbone-carbone ; un groupe alkylène contenant 1 à 7 atomes de carbone ; ou un groupe alkylène contenant 1 à 7 atomes de carbone et étant (a) interrompu avec une liaison éther, une liaison thioéther ou une double liaison carbone-carbone, ou bien (b) fixé au noyau phényle par un atome d'oxygène ou un atome de soufre non oxydé ; ou

lorsque E représente un groupe tétrazolyle ou N-méthyltétrazolyle, B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido ;

ou un dérivé dudit composé dans lequel E représente un groupe carboxyle, choisi entre un ester alkylique inférieur, un ester de (alkyle inférieur)-amino-(alkyle inférieur), un ester de glycolamide, un sel d'addition d'acide d'ester de (alkyle inférieur)-amino-(alkyle inférieur) pharmaceutiquement acceptable et un carboxylate pharmaceutiquement acceptable ;

ou bien un dérivé dudit composé dans lequel E représente un groupe tétrazolyle, choisi entre un sel de métal alcalin ou de métal alcalino-terreux, pharmaceutiquement acceptable, du groupement tétrazolyle.

**2.** Composé suivant la revendication 1, dans lequel X représente un groupe carbonyle.

**3.** Composé suivant la revendication 1, dans lequel X représente un groupe N-R.

**4.** Composé suivant l'une quelconque des revendications 1 à 3, dans lequel Ar représente un groupe cyclohexyle.

**5.** Composé suivant l'une quelconque des revendications 1 à 3, dans lequel Ar représente un groupe phényle.

**6.** Composé suivant l'une quelconque des revendications 1 à 5, dans lequel E représente un groupe carboxyle.

**7.** Composé suivant l'une quelconque des revendications 1 à 5, dans lequel E représente un groupe tétrazolyle.

**8.** Composition pharmaceutique anti-allergique comprenant un composé suivant la revendication 1 et un véhicule pharmaceutiquement acceptable, le composé étant présent en une quantité efficace pour l'obtention d'une réponse anti-allergique chez un mammifère.

**9.** Composé suivant la revendication 1, destiné à être utilisé dans un traitement thérapeutique de l'organisme de l'homme ou d'un animal.

**10.** Composé suivant la revendication 1, destiné à être utilisé dans un traitement thérapeutique de l'organisme de l'homme ou d'un animal, comprenant un traitement destiné à inhiber la bronchoconstriction due à une réponse allergique ou à inhiber la biosynthèse des leucotriènes.

**11.** Utilisation d'un composé suivant la revendication 1 dans un procédé de préparation d'un médicament destiné à inhiber la bronchoconstriction due à une réponse allergique chez un mammifère.

**12.** Utilisation d'un composé suivant la revendication 1, dans un procédé de préparation d'un médicament destiné à inhiber la biosynthèse des leucotriènes chez un mammifère.

**13.** Composé de formule

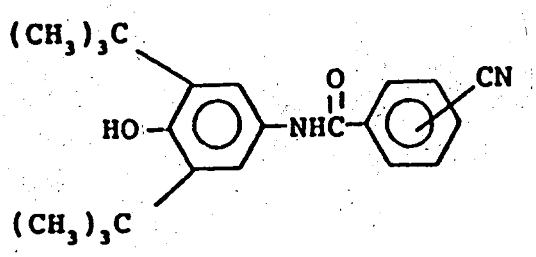

**14.** Composé de formule

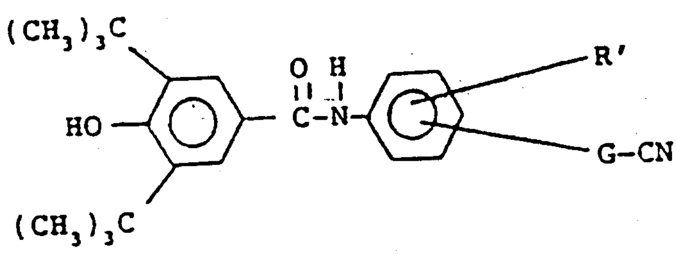

dans laquelle

R' représente l'hydrogène, un halogène, un groupe trifluorométhyle, alkyle inférieur ou alkoxy inférieur ;

G représente une liaison carbone-carbone ou un groupe alkylène ayant 1 à 4 atomes de carbone.

**15.** Procédé de préparation d'un composé de formule

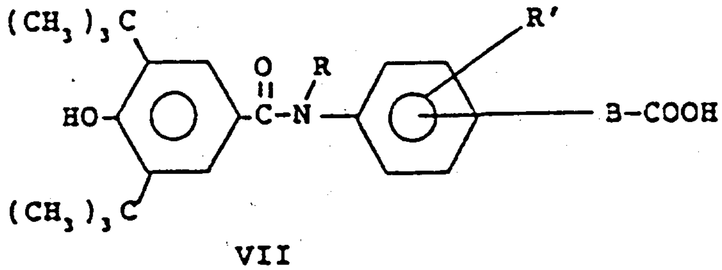

VII

comprenant l'association du chlorure de 3,5-ditertiobutyl-4-hydroxybenzoyle avec une amine de formule

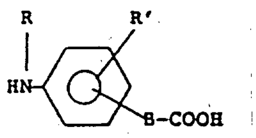

VI

dans un solvant inerte, accompagnée par un chauffage doux si cela est nécessaire ; formules dans lesquelles R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 7 atomes de carbone, ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé.

**16.** Procédé de préparation d'un composé de formule

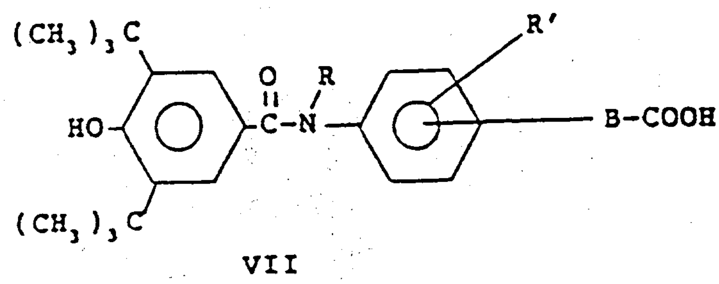

VII

comprenant l'hydrolyse préférentielle du groupe -CN d'un composé de formule

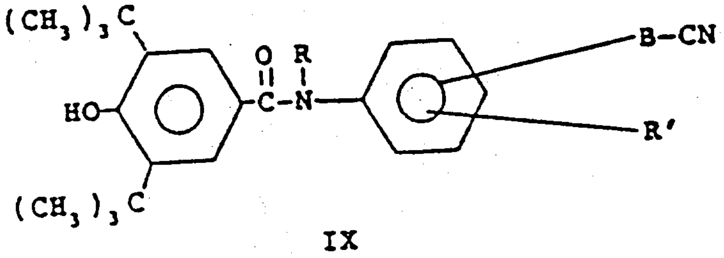

IX

dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 7 atomes de carbone, ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou

un atome de soufre non oxydé.

**17.** Procédé de préparation d'un composé de formule

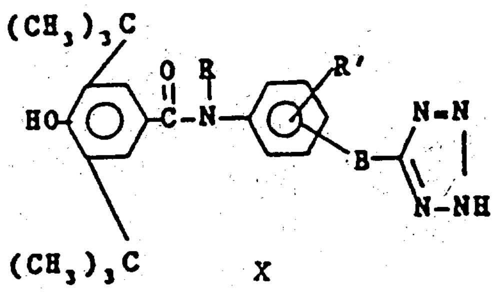

X

comprenant l'association d'un composé de formule

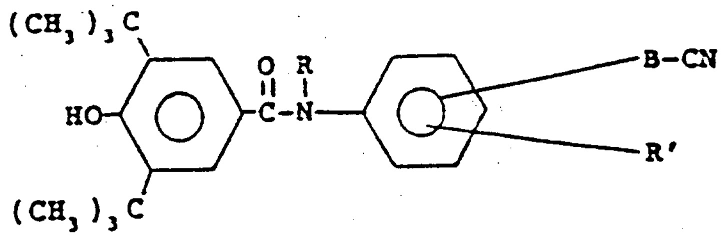

IX

avec l'azoture de sodium, le chlorure d'ammonium et le chlorure de lithium en présence d'un solvant polaire, formule dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido.

**18.** Procédé de préparation d'un composé de formule

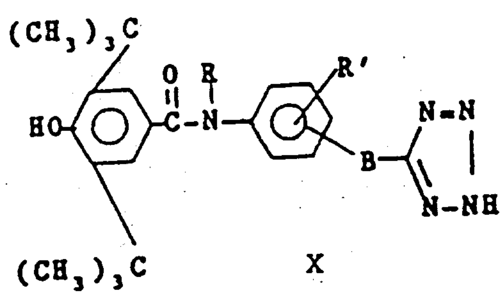

X

comprenant l'association du chlorure de 3,5-ditertiobutyl-4-hydroxybenzolyle et d'un composé de formule

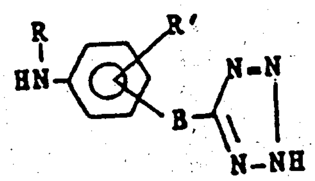

XI

en présence d'un solvant inerte ; formule dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido.

**Revendications pour l'Etat contractant suivant : ES**

**1.** Procédé de préparation d'un composé de formule

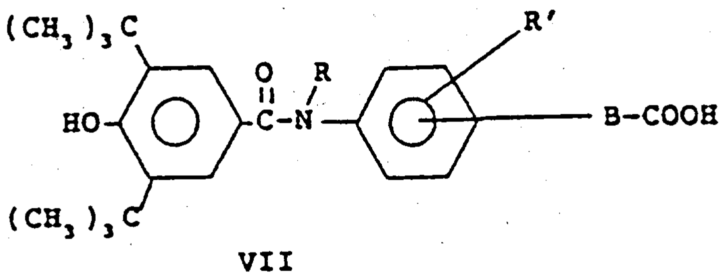

VII

comprenant l'association du chlorure de 3,5-ditertiobutyl-4-hydroxybenzoyle avec une amine de formule

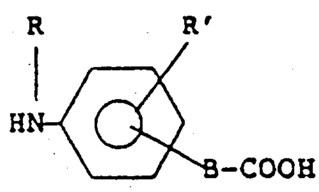

VI

dans un solvant inerte, accompagnée par un chauffage doux si nécessaire ; formule dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 7 atomes de carbone, ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé.

**2.** Procédé de préparation d'un composé de formule

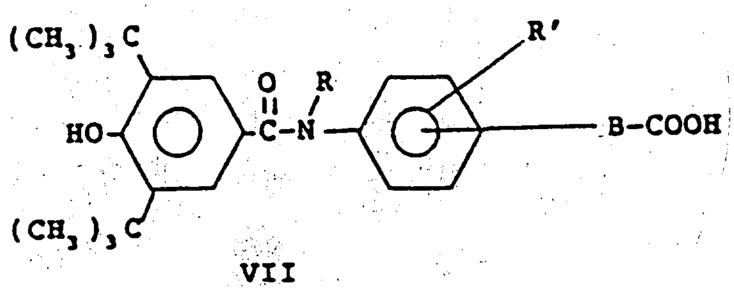

VII

comprenant l'étape consistant à hydrolyser préférentiellement le groupe -CN d'un composé de formule

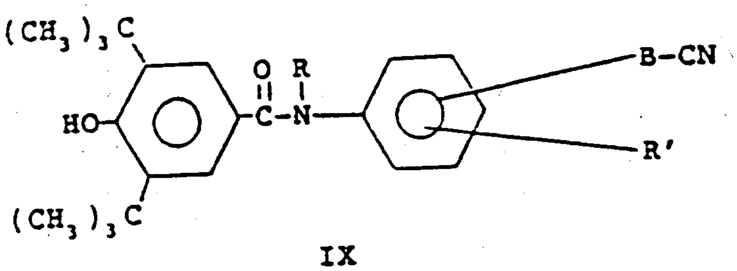

IX

dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 7 atomes de carbone, ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé.

**3.** Procédé de préparation d'un composé de formule

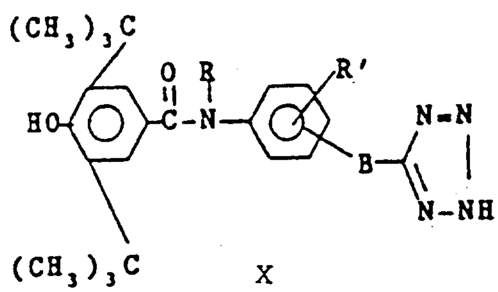

X

comprenant l'association d'un composé de formule

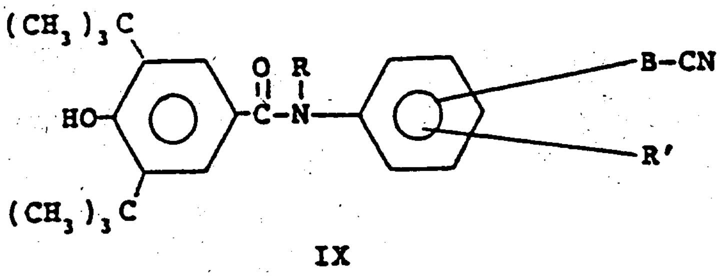

IX

avec l'azoture de sodium, le chlorure d'ammonium et le chlorure de lithium en présence d'un solvant

polaire, formule dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido.

**4.** Procédé de préparation d'un composé de formule

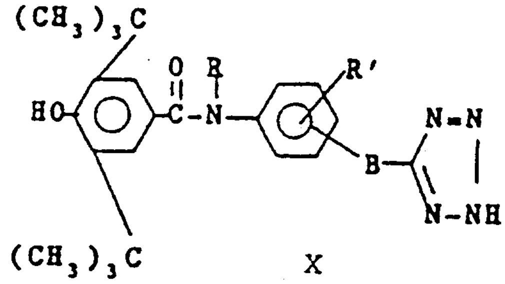

X

comprenant l'association du chlorure de 3,5-ditertiobutyl-4-hydroxybenzoyle et d'un composé de formule

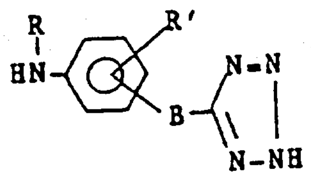

XI

en présence d'un solvant inerte ; formule dans laquelle R représente l'hydrogène ou un groupe alkyle inférieur ; R' représente l'hydrogène, un halogène, un groupe trifluorométhyle, alkyle inférieur ou alkoxy inférieur ; et B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido.

**5.** Utilisation d'un composé de formule

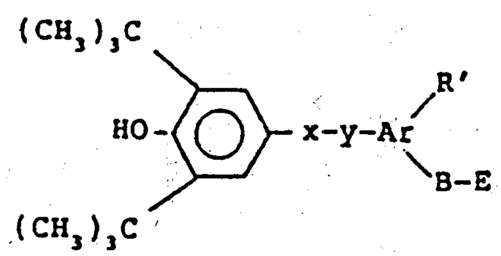

dans laquelle x représente un groupe carbonyle ou N-R, et y représente un groupe carbonyle ou N-R ; sous réserve que, lorsque x représente un groupe carbonyle, y représente un groupe N-R et, lorsque x représente un groupe N-R, y représente un groupe carbonyle ; sous réserve en outre que :

1) lorsque x représente un groupe N-R,

R représente l'hydrogène,

R' représente l'hydrogène,
B représente une liaison carbone-carbone,
Ar représente un groupe phényle ou cyclohexyle et
E représente un groupe tétrazolyle ou carboxyle,

sous réserve que, lorsque Ar représente un groupe cyclohexyle, E représente un groupe carboxyle et soit en position alpha par rapport à la liaison carbonyle-cyclohexyle ; et

2) lorsque x représente un groupe carbonyle,

R représente l'hydrogène ou un groupe alkyle inférieur,
R' représente l'hydrogène, un halogène, un groupe trifluorométhyle, alkyle inférieur ou alkoxy inférieur,
Ar représente un groupe phényle,
E représente un groupe carboxyle, tétrazolyle ou n-méthyltétrazolyle, et
B réponde à la définition suivante :

lorsque E représente un groupe carboxyle, B représente une liaison carbone-carbone ; un groupe alkylène contenant 1 à 7 atomes de carbone ; ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; ou

lorsque E représente un groupe tétrazolyle ou N-méthyltétrazolyle, B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido ;

ou d'un dérivé dudit composé dans lequel E représente un groupe carboxyle, choisi entre un ester alkylique inférieur, un rester de (alkyle inférieur)-amino-(alkyle inférieur), un ester de glycolamide, un sel d'addition d'acide d'ester de (alkyle inférieur)-amino-(alkyle inférieur) pharmaceutiquement acceptable et un carboxylate pharmaceutiquement acceptable ;

ou bien d'un dérivé dudit composé dans lequel E représente un groupe tétrazolyle, choisi entre un sel de métal alcalin et un sel de métal alcalino-terreux pharmaceutiquement acceptables du groupement tétrazolyle dans un procédé de préparation d'un médicament destiné à inhiber la bronchoconstriction due à une réponse allergique chez un mammifère.

**6.** Utilisation d'un composé de formule

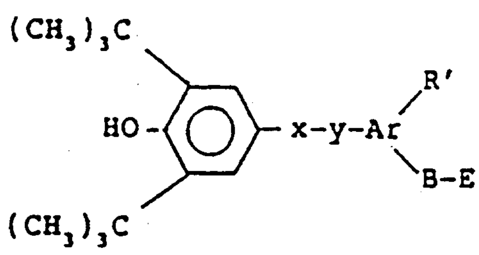

dans laquelle x représente un groupe carbonyle ou N-R, et y représente un groupe carbonyle ou N-R ; sous réserve que, lorsque x représente un groupe carbonyle, y représente un groupe N-R et, lorsque x représente un groupe N-R, y représente un groupe carbonyle ; sous réserve en outre que :

1) lorsque x représente un groupe N-R,

R représente l'hydrogène,
R' représente l'hydrogène,
B représente une liaison carbone-carbone,
Ar représente un groupe phényle ou cyclohexyle et
E représente un groupe tétrazolyle ou carboxyle,

sous réserve que, lorsque Ar représente un groupe cyclohexyle, E représente un groupe carboxyle et soit en position alpha par rapport à la liaison carbonyle-cyclohexyle ; et

2) lorsque x représente un groupe carbonyle,

R représente l'hydrogène ou un groupe alkyle inférieur,

R' représente l'hydrogène, un halogène, un groupe trifluorométhyle, alkyle inférieur ou alkoxy inférieur,
Ar représente un groupe phényle,
E représente un groupe carboxyle, tétrazolyle ou N-méthyltétrazolyle, et
B réponde à la définition suivante :
lorsque E représente un groupe carboxyle, B représente une liaison carbone-carbone ; un groupe alkylène contenant 1 à 7 atomes de carbone ; ou un groupe alkylène contenant 1 à 7 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; ou
lorsque E représente un groupe tétrazolyle ou N-méthyltétrazolyle, B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido ;
ou d'un dérivé dudit composé dans lequel E représente un groupe carboxyle, choisi entre un ester alkylique inférieur, un ester de (alkyle inférieur)-amino-(alkyle inférieur), un ester de glycolamide, un sel d'addition d'acide d'ester de (alkyle inférieur)-amino-(alkyle inférieur) pharmaceutiquement acceptable et un carboxylate pharmaceutiquement acceptable ;
ou bien d'un dérivé dudit composé dans lequel E représente un groupe tétrazolyle, choisi entre un sel de métal alcalin et un sel de métal alcalino-terreux pharmaceutiquement acceptables du groupement tétrazolyle dans un procédé de préparation d'un médicament destiné à inhiber la biosynthèse des leucotriènes chez un mammifère.

**7.** Utilisation d'un composé de formule

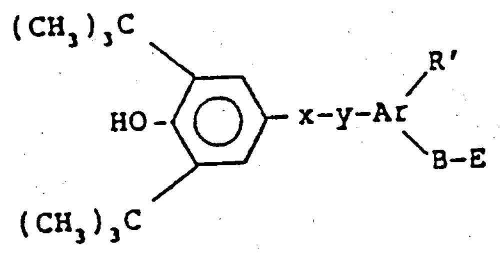

dans laquelle x représente un groupe carbonyle ou N-R, et y représente un groupe carbonyle ou N-R ; sous réserve que, lorsque x représente un groupe carbonyle, y représente un groupe N-R et, lorsque x représente un groupe N-R, y représente un groupe carbonyle ; sous réserve en outre que :
1) lorsque x représente un groupe N-R,
R représente l'hydrogène,
R' représente l'hydrogène,
B représente une liaison carbone-carbone,
Ar représente un groupe phényle ou cyclohexyle et
E représente un groupe tétrazolyle ou carboxyle,
sous réserve que lorsque Ar représente un groupe cyclohexyle, E représente un groupe carboxyle et soit en position alpha par rapport à la liaison carbonyle-cyclohexyle ; et
2) lorsque x représente un groupe carbonyle,
R représente l'hydrogène ou un groupe alkyle inférieur,
R' représente l'hydrogène, un halogène, un groupe trifluorométhyle, alkyle inférieur ou alkoxy inférieur,
Ar représente un groupe phényle,
E représente un groupe carboxyle, tétrazolyle ou N-méthyltétrazolyle, et
B réponde à la définition suivante :
lorsque E représente un groupe carboxyle, B représente une liaison carbone-carbone ; un groupe alkylène contenant 1 à 7 atomes de carbone ; ou un groupe alkylène contenant 1 à 7 atomes de carbone et étant (a) interrompu par une liaison éther, une liaison thioéther ou une double liaison

carbone-carbone, ou bien (b) fixé au noyau phényle par un atome d'oxygène ou un atome de soufre non oxydé ; ou

lorsque E représente un groupe tétrazolyle ou N-méthyltétrazolyle, B représente une liaison carbone-carbone ; un groupe alkylène contenant 1 à 4 atomes de carbone ; un groupe alkylène contenant 1 à 4 atomes de carbone et étant (a) interrompu par une liaison éther, une liaison thioéther ou une double liaison carbone-carbone, ou bien (b) fixé au noyau phényle par un atome d'oxygène ou un atome de soufre non oxydé ; un atome de soufre non oxydé ; ou un groupe carboxamido ;

ou d'un dérivé dudit composé dans lequel E représente un groupe carboxyle, choisi entre un ester alkylique inférieur, un ester de (alkyle inférieur)-amino-(alkyle inférieur), un ester de glycolamide, un sel d'addition d'acide d'ester de (alkyle inférieur)-amino-(alkyle inférieur) pharmaceutiquement acceptable et un carboxylate pharmaceutiquement acceptable ;

ou bien d'un dérivé dudit composé dans lequel E représente un groupe tétrazolyle, choisi entre un sel de métal alcalin et un sel de métal alcalino-terreux pharmaceutiquement acceptables du groupement tétrazolyle dans un procédé de préparation d'un médicament destiné au traitement thérapeutique de l'organisme de l'homme ou d'un animal.